# EUROPÄISCHE PATENTSCHRIFT

(11) **EP 4 658 243 B1**
(45) Veröffentlichungstag und Bekanntmachung des Hinweises auf die Patenterteilung: **25.11.2026**
(21) Anmeldenummer: 24721669.0
(22) Anmeldetag: 25.04.2024
(51) Int. Cl.: A61K 31/045, A61K 9/00, A61K 31/185, A61K 31/702, A61K 36/45, A61K 45/06, A61K 47/06, A61P 13/00, A61P 31/00, A61Q 19/00, A61Q 90/00

(54) **ZUSAMMENSETZUNG AUF BASIS VON PFLANZENEXTRAKTEN UND BISABOLOL**
COMPOSITION ON THE BASIS OF PLANT EXTRACTS AND BISABOLOL
COMPOSITION À BASE D'EXTRAITS VÉGÉTAUX ET DE BISABOLOL

(30) Priorität: 28.06.2023 DE 102023117015; 14.08.2023 DE 102023121682
(43) Veröffentlichungstag der Anmeldung: 10.12.2025
(73) Patentinhaber: Maria Clementine Martin Klosterfrau Vertriebsgesellschaft mbH, 50670 Köln (DE)
(72) Erfinder: OBERBÖRSCH, Stefan, 50670 Köln (DE)
(74) Vertreter: Strehlke, Ingo Kurt
(86) Internationale Anmeldenummer: PCT/EP2024/061377
(87) Internationale Veröffentlichungsnummer: WO 2025/002625

(56) Entgegenhaltungen:
- EP-B1- 2 789 247
- WO-A1-2021/250019
- DE-U1- 202009 008 263
- SIHRA N�HA ET AL: "Nonantibiotic prevention and management of recurrent urinary tract infection", NATURE REVIEWS. UROLOGY, vol. 15, no. 12, 1 December 2018 (2018-12-01), US, pages 750 - 776, XP093085443, ISSN: 1759-4812, DOI: 10.1038/s41585-018-0106-x
- VAHLENSIECK W ET AL: "Prophylaxis of recurrent urinary tract infections", DER UROLOGE, SPRINGER MEDIZIN, HEIDELBERG, vol. 53, no. 10, 9 October 2014 (2014-10-09), pages 1468 - 1475, XP035404336, ISSN: 0340-2592, [retrieved on 20141009], DOI: 10.1007/S00120-014-3562-9
- RIEDL CLAUS R. ET AL: "Hyaluronan treatment of interstitial cystitis/painful bladder syndrome", INTERNATIONAL UROGYNECOLOGY JOURNAL, vol. 19, no. 5, 21 December 2007 (2007-12-21), GB, pages 717 - 721, XP093188955, ISSN: 0937-3462, Retrieved from the Internet <URL:http://link.springer.com/article/10.1007/s00192-007-0515-5/fulltext.html> DOI: 10.1007/s00192-007-0515-5

## Beschreibung

Die vorliegende Erfindung betrifft das Gebiet der Pharma-, Medizin- sowie der Kosmetikprodukte, insbesondere das technische (d. h. medizinischpharmazeutische bzw. kosmetisch-technische) Gebiet der prophylaktischen und/oder therapeutischen Behandlung von Infektionen und/oder entzündlichen Erkrankungen des Urogenitaltraktes bzw. der unterstützenden, vorzugsweise präventiven und/oder kurativen, kosmetischen Intimpflege.

Insbesondere betrifft die vorliegende Erfindung eine Zusammensetzung, insbesondere pharmazeutische und/oder kosmetische Zusammensetzung, insbesondere für die topische Applikation im äußeren Intimbereich.

Darüber hinaus betrifft die vorliegende Erfindung die erfindungsgemäße Zusammensetzung zur Verwendung bei der prophylaktischen und/oder therapeutischen Behandlung von Infektionen und/oder entzündlichen Erkrankungen der Harnwege.

Weiter betrifft die vorliegende Erfindung eine pharmazeutische Zubereitung, insbesondere Creme, Lotion, Cremegel und/oder Gel, vorzugsweise Gel, bevorzugt Arzneimittel oder Medizinprodukt, umfassend eine Zusammensetzung nach der vorliegenden Erfindung sowie eine kosmetische Zubereitung, insbesondere Creme, Lotion, Cremegel und/oder Gel, vorzugsweise Gel, umfassend eine Zusammensetzung nach der vorliegenden Erfindung und deren Verwendung im Intimpflegebereich.

Schließlich betrifft die vorliegende Erfindung eine Dosiervorrichtung, vorzugsweise einen Dosierspender, der eine Zusammensetzung oder eine Zubereitung nach der vorliegenden Erfindung enthält.

Infektionen bzw. entzündliche Erkrankungen des Urogenitaltraktes, welche insbesondere die Harnwege betreffen, sind in der Bevölkerung weit verbreitet. Aufgrund ihrer anatomischen Voraussetzungen sind Frauen von diesen häufiger betroffen als Männer. Synonym wird auch von Harnwegsinfekten gesprochen. Hierunter versteht man eine durch Krankheitserreger verursachte Infektion der ableitenden Harnwege. Dabei kann eine Unterteilung im Hinblick auf die von der Infektion betroffenen Organe bzw. Strukturen in einen sogenannten unteren Harnwegsinfekt und einen oberen Harnwegsinfekt erfolgen. Der untere Harnwegsinfekt stellt meistens das Primärstadium der Infektionskrankheit dar, welches bei entsprechender Schwere der Erkrankung bzw. bei erfolgloser Behandlung zu einer Ausbreitung im Rahmen eines oberen Harnwegsinfektes führen kann. Sofern die Harnblase von dem Infekt betroffen ist, spricht man diesbezüglich von einer, insbesondere bakteriell hervorgerufenen, Zystitis.

In den meisten Fällen kommt ein Harnwegsinfekt über den Aufstieg von Erregern über die Harnröhre zustande, was auch als aszendierende Infektion bezeichnet wird. Als die Harnwegsinfektion verursachende Erreger kommen insbesondere gramnegative Bakterien bzw. Erreger in Betracht, wobei *Escherichia coli* mit 80 % der Fälle als häufigster Erreger von Harnwegsinfekten auftritt. Jedoch können Harnwegsinfekte auch durch grampositive Kokken, Mykoplasmen, Ureaplasmen, Hefen, Chlamydien, Viren oder dergleichen hervorgerufen werden.

Im Allgemeinen entsteht die Infektion häufig dadurch, dass Erreger zur äußeren Harnröhrenöffnung gelangen und von dort die Harnröhre hinauf in die Harnblase wandern, wo sie beispielsweise zu der zuvor beschriebenen Zystitis führen können. Eine wichtige Rolle im Verlauf der Infektion spielt die Kontaktaufnahme und Adhäsion der Erreger mit dem Zielgewebe, wie beispielsweise dem Epithel der Harnwege, wobei bestimmte Oberflächenmoleküle der Erreger diesbezüglich von hoher Bedeutung sind.

Die mit der Harnwegsinfektion einhergehenden Komplikationen sind umfangreich und können mitunter schwerwiegend, zumindest aber belastend für das betroffene Individuum sein: So führt beispielsweise eine Infektion der Harnröhre selbst zu Schmerzen beim Urinieren oder löst auch einen unangenehmen Juckreiz aus. Zudem ist der Harnfluss oftmals vermindert.

Aufgrund der zum Teil nur geringen Spezifität bzw. Ausprägung der Symptome ist die Diagnose von Harnwegsinfekten nicht immer einfach bzw. eindeutig. Eine weitere Problematik bei entzündlichen Erkrankungen der Harnwege liegt darin, dass häufig Rezidive auftreten, wobei in diesem Zusammenhang auch von chronisch rezidivierenden Harnwegsinfekten gesprochen wird. Chronisch rezidivierende Harnwegsinfekte stellen dabei insbesondere aufgrund der damit einhergehenden teilweise schmerzhaften Symptomatik eine hohe Belastung für die betroffenen Individuen dar.

Zur therapeutischen Behandlung von Harnwegsinfekten wird im Allgemeinen in erster Linie eine Eliminierung der Erreger bzw. Krankheitskeime abgestellt, wobei vor allem die Gabe von Antibiotika häufig in Betracht gezogen wird. Die diesbezüglich mit der Verabreichung von Antibiotika üblicherweise einhergehenden Komplikationen und Nebenwirkungen sind jedoch als nachteilig zu bewerten, beispielsweise u.a. allergische Reaktionen, Hypoglykämien oder auch durch Antibiotika induzierte Hautschädigungen. Zudem kann die häufige Gabe von Antibiotika die Resistenzbildung fördern, was zur Ineffektivität des Therapieansatzes führen kann.

Als präventive bzw. prophylaktische Maßnahmen sind im Stand der Technik die Einnahme von harntreibenden Präparaten bzw. Getränken, bspw. Tees oder dergleichen bekannt. In diesem Zusammenhang bekannte sowie geeignete Wirk- bzw. Inhaltsstoffe entsprechender Präparate basieren bspw. oft auf Brennnessel und deren Extrakten. Die Gabe bzw. Einnahme Brennnessel-basierter Präparate als Tee oder dergleichen erreicht zudem eine Erhöhung der Trinkmenge, was eine verbreitete Allgemeinmaßnahme zur Vorbeugung von Harnwegsinfekten darstellt.

Ein anderer Ansatz im Stand der Technik verfolgt das Prinzip, die Interaktion zwischen Erreger und betroffenem Organ bzw. betroffener Struktur, etwa der Harnröhre oder dem Blasenepithel, zu minimieren bzw. zu verhindern oder zu unterbinden. Zu diesem Zwecke werden bspw. pflanzliche Präparate aus Moosbeere bzw. Kranbeere ("Cranberry") in Form von Monopräparaten zur oralen Applikation eingesetzt. Die orale Applikation von Wirk- bzw. Inhaltsstoffen kann jedoch mit einer verminderten Wirkeffektivität einhergehen, da die Präparate stets zunächst den Magen passieren müssen, was das Risiko birgt, dass ein Teil der Wirk- bzw. Inhaltsstoffe dort vorzeitig abgebaut wird, bevor der eigentliche Wirkort erreicht ist bzw. eine Resorption des Wirkstoffes erfolgen konnte.

Entsprechend existieren im Stand der Technik auch Ansätze, die von topisch anwendbaren Formulierungen ausgehen, welche direkt im Intimbereich angewendet werden. Diese Zusammensetzungen, die beispielsweise in Form von Cremes oder Gelen vorliegen, enthalten u.a. bspw. Mannose, welcher antibakterielle Eigenschaften zugeschrieben werden (d.h. eine die Ausbreitung und/oder das Ansiedeln von Bakterien hemmende bzw. beschränkende Wirkung). Speziell soll Mannose die Interaktion von Erregern wie *E. coli* mit dessen Zielepithel vermindern bzw. unterbinden können, indem die Mannose als FimH-Antagonist wirkt. So ist Mannose in der Lage, an das FimH-Protein auf den bakteriellen Pili zu binden und so das Anheften und das Eindringen der Bakterien in die Zellen der Blasenschleimhaut zu verhindern. Jedoch bindet Mannose FimH-Proteine vergleichsweise schwach, was sich nachteilig auf die Wirksamkeit bzw. Effektivität der Formulierungen auswirkt. Eine Steigerung der Wirksamkeit topisch anzuwendender Formulierung ist vor diesem Hintergrund entsprechend wünschenswert.

Insgesamt führen die im Stand der Technik vorgesehenen medikamentösen Ansätze zur Vorbeugung bzw. Behandlung von Harnwegsinfekten also nicht immer zu einem ausreichenden Therapieerfolg bzw. weisen oftmals auch gravierende Nebenwirkungen auf.

Die EP 2 789 247 B1 betrifft einen Extrakt, welcher Kaffeesäure, Sinapinsäure sowie gegebenenfalls Polyphenole enthalten soll, und zwar mit der Maßgabe, dass Kaffeesäure einerseits sowie Sinapinsäure andererseits in einem speziellen Gewichtsverhältnis in dem Extrakt vorliegen sollen.

Weiterhin betrifft die DE 20 2009 008 263 U1 eine Zusammensetzung, insbesondere bei Infektionen bzw. entzündlichen Erkrankungen des Urogenitaltrakts, wobei die Zusammensetzung Moosbeere und Meerrettich enthalten soll.

Die WO 2021/250019 A1 betrifft eine Zusammensetzung zur Anwendung in einem Verfahren zur Prophylaxe bzw. Behandlung von Harnwegsinfektionen, wobei die Zusammensetzung in Form eines Gels vorliegen und 1 bis 50 % Mannose enthalten soll.

Das Dokument gemäß Sihra N. et al. "Nonantibiotic prevention and management of recurrent urinary tract infection", Nature Reviews Urology, Dezember 2018, Vol. 15, Seiten 750 bis 776, betrifft Ausführungen zu möglichen nicht-anibiotischen Maßnahmen und Behandlungen von Harnwegsinfektionen.

Weiterhin betrifft das Dokument gemäß Vahlensieck W. et al., "Prophylaxis of recurrent urinary tract infections", Der Urologe, Springer Medizin, Heidelberg, Band 53, Nr. 10, Seiten 1468 bis 1475, Ausführungen zur Prophylaxe von Harnwegsinfektionen, wobei eine Übersicht zu diesbezüglichen Maßnahmen gegeben wird.

Das Dokument gemäß Riedl, C. et al., "Hyaluronan treatment of interstitial cystitis/painful bladder syndrome", International Urogynecology Journal, Band 19, Nr. 5, 21. Dezember 2007, Seiten 717 bis 721, betrifft eine Studie zur Wirksamkeit einer intravesikalen Hyaluronan-Therapie bei interstitieller Zystitis bzw. schmerzhaftem Blasensyndrom (IC-PBS).

Vor diesem Hintergrund besteht - insbesondere auch aufgrund der großen Verbreitung und des häufig rezidivierenden Auftretens von Harnwegsinfektionen - ein großer Bedarf, geeignete Zusammensetzungen sowie Therapieformen zur Prävention bzw. Behandlung von Harnwegsinfekten bereitzustellen.

Insgesamt besteht im Stand der Technik also weiterhin ein Bedarf an der Bereitstellung von effizienten Konzepten zur prophylaktischen bzw. therapeutischen Behandlung von insbesondere rezidivierenden Infektionen und/oder entzündlichen Erkrankungen des Urogenitaltraktes und/oder von effizienten Konzepten zur unterstützenden, insbesondere präventiven und/oder kurativen, kosmetischen Pflege bzw. Förderung der Gesundheit und des Gleichgewichtes des Intimbereichs, insbesondere wobei das Konzept eine effiziente bzw. vergleichsweise hohe Wirksamkeit mit einer guten bzw. insbesondere im Alltag leichte handhabbaren Anwendbarkeit kombiniert.

Vor dem Hintergrund des zuvor geschilderten Standes der Technik liegt der vorliegenden Erfindung somit die Aufgabe zugrunde, ein effizientes Konzept bzw. eine diesbezügliche Zusammensetzung bereitzustellen, welche(s) eine effiziente Behandlung von insbesondere rezidivierenden Infektionen und/oder entzündlichen Erkrankungen des Urogenitaltraktes und/oder eine, insbesondere präventive und/oder kurative, kosmetische Pflege bzw. Förderung der Gesundheit des Intimbereichs ermöglicht, wobei in diesem Zusammenhang die zuvor angeführten Nachteile des Standes der Technik überkommen oder zumindest abgeschwächt werden sollen.

Insbesondere liegt der vorliegenden Erfindung die Aufgabe zugrunde, entsprechende Zusammensetzungen bzw. auch Zubereitungen bereitzustellen, welche im Rahmen der Behandlung von insbesondere rezidivierenden Infektionen und/oder entzündlichen Erkrankungen des Urogenitaltraktes und/oder bei der unterstützenden, insbesondere präventiven und/oder kurativen, kosmetischen Intimpflege eine hervorragende Wirkeffizienz sowie eine unkomplizierte Applikation bei gleichzeitig guter Verträglichkeit sowie auch einer angenehmen sensorischen Empfindung aufweisen.

In vollkommen überraschender Weise hat die Anmelderin nunmehr gefunden, dass anhand des dem der vorliegenden Erfindung zugrundeliegenden erfinderischen Konzepts eine für die topische Applikation im äußeren Intimbereich besonders geeignete, und zwar eine insbesondere im Hinblick auf die Verwendung bei der prophylaktischen und/oder therapeutischen Behandlung von Infektionen und/oder entzündlichen Erkrankungen des Urogenitaltraktes, insbesondere der Harnwege, geeignete, Zusammensetzung, erhalten werden kann, die sich sowohl für den Einsatz als Kosmetikprodukt als auch als Medikament, zumindest zur Linderung des Verlaufs von Urogenitaltraktinfektionen, vorteilhaft anbietet.

Zur Lösung der zuvor geschilderten Problemstellung schlägt die vorliegende Erfindung somit - gemäß einem **ersten** Aspekt der vorliegenden Erfindung - eine Zusammensetzung, insbesondere pharmazeutische und/oder kosmetische Zusammensetzung, gemäß Anspruch 1, vor. Jeweils vorteilhafte Weiterbildungen und Ausgestaltungen dieses Erfindungsaspekts sind Gegenstand der diesbezüglichen Unteransprüche.

Weiterer Gegenstand der vorliegenden Erfindung - gemäß einem **zweiten** Aspekt der vorliegenden Erfindung - ist eine Zusammensetzung nach der vorliegenden Erfindung zur Verwendung bei der prophylaktischen und/oder therapeutischen Behandlung von Infektionen und/oder entzündlichen Erkrankungen der Harnwege gemäß dem diesbezüglichen unabhängigen Anspruch.

Wiederum weitere Gegenstände der vorliegenden Erfindung - gemäß einem **dritten**, **vierten** und **fünften** Aspekt der vorliegenden Erfindung - sind eine pharmazeutische Zubereitung bzw. eine kosmetische Zubereitung, jeweils umfassend eine Zusammensetzung nach der vorliegenden Erfindung, sowie deren Verwendung im Intimpflegebereich gemäß den diesbezüglichen unabhängigen Ansprüchen.

Schließlich Gegenstand der vorliegenden Erfindung - gemäß einem **sechsten** Aspekt der vorliegenden Erfindung - ist eine Dosiervorrichtung, vorzugsweise ein Dosierspender, enthaltend eine Zusammensetzung oder eine Zubereitung je nach der vorliegenden Erfindung, gemäß dem diesbezüglichen unabhängigen Anspruch.

Es versteht sich von selbst, dass Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von dem angegebenen Bereich bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Ferner ist bei allen nachstehend genannten relativen bzw. prozentualen, insbesondere gewichtsbezogenen Mengenangaben zu beachten, dass diese Angaben im Hinblick auf das herangezogene Bezugssystem (z. B. Darreichungsform) vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Darüber hinaus gilt für die Beschreibung der vorliegenden Erfindung, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Insbesondere gilt für die die Erfindung charakterisierenden Merkmale, dass sämtliche Kombinationsmöglichkeiten dieser Merkmale als offenbart gelten, wobei Ausführungsformen vergleichbarer bzw. entsprechender Präferenz der verschiedenen Merkmale in ihrer Kombination bevorzugt sind (z.B. Mengen bzw. Mengenbereiche der betreffenden Wirk- und Inhaltsstoffe gleicher Präferenz oder dergleichen).

Dabei gilt insbesondere auch, dass für die nachfolgend angeführten, die verschiedenen Inhaltsstoffe, insbesondere Wirkstoffe, der erfindungsgemäßen Zusammensetzung oder dergleichen betreffenden Mengenangaben, insbesondere relativen Mengenangaben oder absoluten Mengenangaben gleicher Präferenz bzw. gleicher Bevorzugungsebene, jeweilige und die verschiedenen Inhaltsstoffe, insbesondere Wirkstoffe, betreffende Kombinationen mit entsprechender Präferenz bzw. Bevorzugung mitoffenbart sind. Ebenfalls sind auch sämtliche anderweitige Kombinationen (d.h. Kombinationen auf Basis unterschiedlicher Präferenzen bzw. unterschiedlicher Bevorzugungsebenen) mitoffenbart.

Dies vorausgeschickt, wird die vorliegende Erfindung nunmehr nachfolgend im Detail erläutert:
Gegenstand der vorliegenden Erfindung - gemäß einem **ersten** Aspekt der vorliegenden Erfindung - ist eine Zusammensetzung, insbesondere pharmazeutische und/oder kosmetische Zusammensetzung, insbesondere für die topische Applikation im äußeren Intimbereich, vorzugsweise zur Verwendung bei der prophylaktischen und/oder therapeutischen Behandlung von Infektionen und/oder entzündlichen Erkrankungen des Urogenitaltraktes, insbesondere der Harnwege,
wobei die Zusammensetzung in Kombination und in jeweils wirksamen, insbesondere in jeweils pharmazeutisch und/oder kosmetisch wirksamen, Mengen
   a) Bisabolol, insbesondere alpha-Bisabolol (Levomenol),
   b) mindestens einen Extrakt von Gewöhnlicher Moosbeere (*Vaccinium oxycoccos*),
      wobei der Extrakt von Gewöhnlicher Moosbeere (*Vaccinium oxycoccos*) ein Flüssigextrakt ist,
      wobei der Flüssigextrakt mindestens einen Trockenextrakt von Gewöhnlicher Moosbeere (*Vaccinium oxycoccos*) und einen Lösevermittler enthält,
      wobei der Trockenextrakt von Gewöhnlicher Moosbeere (*Vaccinium oxycoccos*) ein Droge/Extrakt-Verhältnis (DEV) in einem Bereich von 20 : 1 bis 1 : 10, bezogen auf den Trockenextrakt, aufweist,
   c) mindestens einen Extrakt von Preiselbeere (*Vaccinium vitis-idaea* L*.*),
      wobei der Extrakt von Preiselbeere (*Vaccinium vitis-idaea* L.) ein Flüssigextrakt ist,
      wobei der Flüssigextrakt mindestens einen Trockenextrakt von Preiselbeere (*Vaccinium vitis-idaea* L.) und einen Lösevermittler enthält,
      wobei der Trockenextrakt von Preiselbeere (*Vaccinium vitis-idaea* L.) ein Droge/Extrakt-Verhältnis (DEV) in einem Bereich von 75 : 1 bis 35 : 1, bezogen auf den Trockenextrakt, aufweist und
      wobei der Trockenextrakt von Preiselbeere (*Vaccinium vitis-idaea* L.) einen Proanthocyanidin-Gehalt in einem Bereich von 0,01 bis 5 Gew.-%, bezogen auf die Gesamtzusammensetzung des Extrakts, aufweist,
   d) Mannose, insbesondere D-Mannose,
umfasst;
wobei die Zusammensetzung, bezogen auf die Gesamtzusammen-setzung,
die Komponente a) in Mengen im Bereich von 0,01 Gew.-% bis 1,25 Gew.-% enthält,
die Komponente b) in Mengen im Bereich von 0,1 Gew.-% bis 5 Gew.-% enthält,
die Komponente c) in Mengen im Bereich von 0,1 Gew.-% bis 5 Gew.-% enthält,
die Komponente d) in Mengen von weniger als 1 Gew.-% enthält.

Auf Grundlage der erfindungsgemäßen Zusammensetzung und speziell der besonderen Kombination der Komponenten a) bis d), welche in der Zusammensetzung umfasst ist, gelang es in völlig überraschender Weise, eine hocheffiziente sowie -effektive Inhalts- bzw. Wirkstoffkombination bzw. Kombination physiologisch aktiver Inhaltsstoffe in einer Zusammensetzung bereitzustellen, welche nachweislich vorteilhaft dazu beiträgt, die prophylaktische bzw. therapeutische Behandlung von Infektionen des Urogenitaltraktes zu unterstützen sowie im Rahmen der Intimpflege einen wirkungsvollen Beitrag zur Prävention von Harnwegsinfekten und zur Gesunderhaltung des Intimbereichs zu leisten.

Die besondere Wirksamkeit erfindungsgemäßer Zusammensetzungen ist in der speziellen Kombination der Komponenten a) bis d) begründet, d.h. der besonderen Mischung zweier pflanzlicher Extrakte von Pflanzen der Gattung Vaccinium, Bisabolol, insbesondere alpha-Bisabolol (Levomenol), und Mannose, insbesondere D-Mannose.

Wie bereits eingangs erwähnt, zeichnet sich Mannose durch eine Aktivität als FimH-Antagonist aus und kann so einen Beitrag dazu leisten, die Interaktion von *E. coli* mit dem Epithel der ableitenden Harnwege, dem Urothel, zu stören bzw. zu unterbinden. Diese Eigenschaft von Mannose wird im Rahmen der vorliegenden Erfindung vorteilhaft positiv komplementiert durch die in der erfindungsgemäßen Zusammensetzung eingesetzten Extrakte von Gewöhnlicher Moosbeere (*Vaccinium oxycoccos*) bzw. Preiselbeere (*Vaccinium vitis-idaea*) sowie durch das enthaltene Bisabolol.

Bisabolol zeichnet sich dabei durch entzündungshemmende, antimikrobielle sowie hautberuhigende Eigenschaften aus und weist zudem auch potenziell antioxidative Eigenschaften auf. Außerdem kann es krampflösend bzw. schmerzstillend wirken, was im Falle der Anwendung der erfindungsgemäßen Zusammensetzung bei einer akuten Zystitis vorteilhaft als lindern bzw. mildernd empfunden werden kann. Hinsichtlich der antimikrobiellen Wirksamkeit bzw. Wirkung ist Bisabolol dazu in der Lage, bestimmte Toxine, die von Bakterien abgegeben werden, zu neutralisieren und so die Pathogenität der Erreger zu reduzieren.

Bisabolol verbessert darüber hinaus vorteilhaft auch die perkutane Absorption und wirkt vorteilhaft als Penetrationsverstärker in topischen Formulierungen, was entsprechend die Absorptionsfähigkeit der übrigen Zusammensetzungskomponenten unter der Haut vorteilhaft erhöht und so die Wirksamkeit der Einzelkomponenten der erfindungsgemäßen Zusammensetzung verstärken und positiv beeinflussen kann.

Die in der erfindungsgemäßen Zusammensetzung eingesetzten Pflanzenextrakte von Gewöhnlicher Moosbeere (*Vaccinium oxycoccos*) bzw. Preiselbeere (*Vaccinium vitis-idaea*) zeichnen sich vorteilhaft durch unter anderem hohe Anteile an Tanninen, Proanthocyanidinen, Anthocyanidinen, Flavonoiden, Benzoesäure, Vitamin C, Natrium, Phosphor und Kalium aus. Insbesondere den in den Pflanzenextrakten enthaltenen Tanninen, speziell Proanthocyanidinen, wird eine antibakterielle Wirksamkeit (d.h. eine bakterienblockierende Wirkung) zugeschrieben, welche sich darauf stützt, dass Proanthocyanidine das Anhaften von Bakterien, insbesondere von *E. coli,* an uroepithelialen Zellen, speziell mittels der P-Fimbrien, hemmen bzw. verhindern können, wodurch die Ausbreitung der Keime in den Urogenitaltrakt insgesamt stark eingeschränkt bzw. minimiert werden kann.

Ähnlich wie Mannose unterbinden die Proanthocyanidine der eingesetzten Pflanzenextrakte von Gewöhnlicher Moosbeere (*Vaccinium oxycoccos*) bzw. Preiselbeere *(Vaccinium vitis-idaea)* also die Interaktion von Krankheitserregern mit Zellen des Urothel, wobei als besonders vorteilhaft hervorzuheben ist, dass Mannose und die Proanthocyanidine der eingesetzten Pflanzenextrakte von Gewöhnlicher Moosbeere (*Vaccinium oxycoccos*) bzw. Preiselbeere (*Vaccinium vitis-idaea*) unterschiedliche Oberflächenproteine der Krankheitserreger, insbesondere von *E. coli,* adressieren, so dass ein erweitertes Inhibitionsspektrum erreicht wird und auf mehrfache gleich Weise die Adhäsion bzw. Interaktion von *E. coli* mit den uroepithelialen Zellen des Urogenitaltraktes erschwert bzw. inhibiert werden kann. So wird eine deutliche und insbesondere überproportionale Steigerung der Wirksamkeit bzw. Effektivität der erfindungsgemäßen Zusammensetzung auf Basis der Kombination von Mannose, Bisabolol und den Proanthocyanidinen aus den eingesetzten Pflanzenextrakten von Gewöhnlicher Moosbeere (*Vaccinium oxycoccos*) bzw. Preiselbeere (*Vaccinium vitis-idaea*), insbesondere gegenüber vergleichbaren, Monopräparaten, erreicht.

Die kombinierte sowie gesteigerte antagonistische Wirkung der Komponenten a) bis c) der erfindungsgemäßen Zusammensetzung wird dabei positiv komplementiert durch die vorgenannten vorteilhaften Wirkungen von Bisabolol. Letztlich kann so in insgesamt synergistischer Weise eine deutliche Steigerung der Wirksamkeit und Effektivität der erfindungsgemäßen Zusammensetzung für die bevorzugt vorgesehenen Verwendungen dieser beobachtet werden. Diese deutliche sowie vor allem auch überraschende Steigerung der Wirksamkeit in Bezug auf die antibakterielle Wirkung der erfindungsgemäßen Zusammensetzung bei gleichzeitig auch ausgeprägten hautpflegenden sowie feuchtigkeitsspendenden Eigenschaften der erfindungsgemäßen Zusammensetzung kann dabei vor allem auch dann beobachtet werden, wenn in der Zusammensetzung vergleichsweise geringen Mengen der Komponenten a) bis d) eingesetzt werden, d.h. selbst bei einer Reduktion des Wirkstoffanteils bzw. des Anteils aktiver Inhaltsstoffe im Vergleich zu Zusammensetzungen aus dem Stand der Technik wird eine Steigerung der Wirksamkeit der erfindungsgemäßen Zusammensetzung gegenüber den Zusammensetzungen des Standes der Technik beobachtet.

Insbesondere für die Komponente Mannose ist dabei im Stand der Technik beobachtet worden, dass erst sehr hohe Konzentrationen von Mannose in einem Präparat eine zuverlässige und ausgeprägte antibakterielle Wirkung einer topisch anzuwendenden Zusammensetzung herbeiführen. Vor diesem Hintergrund erscheint eine Dosisverringerung entsprechend abwegig und wenig zielführend für die Bereitstellung einer Zusammensetzung mit verbesserten Wirkeigenschaften.

Im Hinblick auf diesen Umstand und die sich ergebende Tendenz, dass eher bzw. vor allem hohe Mannose-Konzentrationen zu einer hinreichenden, insbesondere antibakteriellen, Wirksamkeit entsprechender Zusammensetzungen führen, ist es daher umso überraschender, dass es im Rahmen der vorliegenden Erfindung und vor allem auf Grund der erfindungsgemäßen Kombination von Mannose, Bisabolol und Pflanzenextrakten von Gewöhnlicher Moosbeere (*Vaccinium oxycoccos*) bzw. Preiselbeere (*Vaccinium vitis-idaea*) möglich ist, sowohl hochwirksame als auch insbesondere niedrigkonzentrierte Zusammensetzungen bereitstellen, wobei diese Zusammensetzungen vorteilhaft mit als sehr positiv zu bewertenden Anwendungseigenschaften für die vorgesehenen Verwendungen einhergehen.

Es ist die besondere Erkenntnis der Anmelderin, dass die antibakteriellen Effekte der Komponenten a) bis d) der erfindungsgemäßen Zusammensetzung einander ergänzen und hierbei insbesondere synergistisch zusammenwirken. Die synergistische Wirkung ergibt sich dabei - ohne an diese Theorie gebunden sein zu wollen -aus den unterschiedlichen, einander im Grunde jedoch recht ähnlichen und vor allem ergänzenden, antagonistischen Wirkungen von Mannose und den Proanthocyanidinen der eingesetzten Pflanzenextrakte von Gewöhnlicher Moosbeere (*Vaccinium oxycoccos*) bzw. Preiselbeere (*Vaccinium vitis-idaea*), welche jeweils gegen spezifische Oberflächenproteine von insbesondere *E. coli* gerichtet sind. Der sich ergebende kombinatorische Effekt, dass die Adhäsion bzw. Interaktion von *E. coli* mit Zellen des Urothel gleich mehrfach erschwert bzw. inhibiert wird, scheint deutlich verstärkt zu werden durch die antimikrobiellen sowie insbesondere die penetrationsverstärkenden und entzündungsmildernden Eigenschaften von Bisabolol. Letztlich kann so insgesamt eine deutlich gesteigerte Wirksamkeit gegenüber Präparaten, die ausschließlich Mannose oder Kombinationen von Mannose mit nur einem weiteren Wirkstoff enthalten, erreicht werden, insbesondere wobei gleichzeitig deutlich geringere Mengenanteile, insbesondere von Mannose, in der erfindungsgemäßen Zusammensetzung eingesetzt werden können.

Über die antibakteriellen Eigenschaften hinaus zeichnen sich die Komponenten a), b) und c) bzw. Bisabolol und die Pflanzenextrakten von Gewöhnlicher Moosbeere (*Vaccinium oxycoccos*) bzw. Preiselbeere (*Vaccinium vitis-idaea*) auch durch hautpflegende Eigenschaften aus. Durch die antioxidative Wirkung der Komponenten kann die Hautalterung positiv beeinflusst bzw. können Zellschädigungen vorbeugt werden. Die Haut wird gesund erhalten und strukturell gestärkt, was sie widerstandfähiger gegen das Eindringen von Erregern wie *E. coli* macht. Die penetrationsverstärkenden Eigenschaften von Bisabolol unterstützen die vorgenannten Wirkeffekte vorteilhaft, indem eine leichtere und insbesondere effizientere Aufnahme der Komponenten der erfindungsgemäßen Zusammensetzung in die Haut erreicht werden kann.

Der Begriff "Zusammensetzung", wie er im Rahmen der vorliegenden Erfindung verwendet wird, ist dabei breit zu verstehen und meint bzw. umfasst neben pharmazeutischen Präparaten bzw. Pharmazeutika und Arzneimitteln als solchen auch sogenannte Medizinprodukte sowie auch Kosmetika.

Der Begriff "Pharmazeutische Zusammensetzung bzw. Zubereitung" meint bzw. umfasst eine Zubereitung aus Stoffen, die "zur Heilung oder zur Verhütung menschlicher Krankheiten" bestimmt sind oder sich zur Beeinflussung physiologischer Funktionen eignen oder eine medizinische Diagnose ermöglichen.

Der Begriff "Kosmetische Zusammensetzung bzw. Zubereitung " meint bzw. umfasst eine Zubereitung aus Stoffen, die "im Allgemeinen dafür vorgesehen sind, äußerlich mit dem menschlichen Körper (Haut, Nägel, Haare), den Zähnen und den Mundschleimhäuten in Berührung zu kommen, vorliegend im Speziellen mit der Haut, insbesondere des Intimbereichs wobei die Zubereitung bzw. Zusammensetzung im Allgemeinen dazu dient bzw. dienen kann, den Körper zu reinigen, zu parfümieren, dessen Aussehen zu verändern, ihn zu schützen, in gutem Zustand zu halten oder den Körpergeruch zu beeinflussen.

Der Begriff "topische Applikation" meint bzw. umfasst die lokale Verabreichung von Medikamenten bzw. den lokalen Auftrag eines Kosmetikums, insbesondere deren äußerliche örtliche Applikation, vorzugsweise auf der Haut.

Der Begriff "Extrakt" meint bzw. umfasst im Rahmen der Erfindung, insbesondere in Zusammenhang mit pharmazeutischen Zwecken, Zubereitungen, die mittels Extraktion ("Ausziehung") aus pharmazeutischen Drogen gewonnen werden, welche entweder direkt als Arzneimittel verwendet werden oder zu solchen weiterverarbeitet werden können. Im Falle von Pflanzenextrakten stellen Pflanzen oder Teile davon in verarbeitetem oder unverarbeitetem Zustand das Ausgangsmaterial dar.

Nachfolgend wird die vorliegende Erfindung weiterführend und im Detail beschrieben, insbesondere im Hinblick auf weitere bevorzugte Ausgestaltungen bzw. bevorzugte Ausführungsformen.

Es hat sich im Rahmen der vorliegenden Erfindung bewährt, wenn die Zusammensetzung, bezogen auf die Gesamtzusammensetzung, die Komponente d) bzw. Mannose, insbesondere D-Mannose, in Mengen von weniger als 1 Gew.-% (< 1 Gew.-%), insbesondere in Mengen von weniger als 0,95 Gew.-% (< 0,95 Gew.-%), vorzugsweise in Mengen von weniger als 0,9 Gew.-% (< 0,9 Gew.-%), bevorzugt in Mengen von weniger als 0,85 Gew.-% (< 0,85 Gew.-%), enthält und/oder in Mengen im Bereich von 0,1 Gew.-% bis 0,99 Gew.-%, insbesondere in Mengen im Bereich von 0,2 Gew.-% bis 0,95 Gew.-%, vorzugsweise in Mengen im Bereich von 0,4 Gew.-% bis 0,9 Gew.-%, bevorzugt in Mengen im Bereich von 0,5 Gew.-% bis 0,85 Gew.-%, enthält.

Vorteilhaft können im Rahmen der vorliegenden Erfindung also hochwirksame bzw. -effiziente Zusammensetzungen mit als vorteilhaft zu bewertenden antibakteriellen sowie auch hautpflegenden Eigenschaften erhalten werden, wobei in diesen Zusammensetzungen nur sehr geringe Mengen von Mannose, insbesondere D-Mannose, vorhanden sein können, ohne dass die Wirksamkeit bzw. Effizienz der erfindungsgemäßen Zusammensetzung hierdurch auf eine nachteilige Weise gesenkt wird.

Wie bereits eingangs beschrieben, wirken die Komponenten a) bis d) der erfindungsgemäßen Zusammensetzung bzw. Bisabolol, die Pflanzenextrakte von gewöhnlicher Moosbeere und Preiselbeere sowie Mannose in synergistischer Weise zusammen, was es insbesondere gestattet, den Mengenanteil von Mannose vergleichsweise gering zu halten, ohne dass der antibakterielle Effekt von Mannose hierdurch verringert bzw. geschmälert würde. Dies ist umso erstaunlicher, da im Stand der Technik bislang die Auffassung vertreten ist, dass erst vergleichsweise hohe Konzentrationen von Mannose in einer Zusammensetzung, die in vergleichbarer Weise wie im Rahmen der vorliegenden Erfindung vorzugsweise vorgesehen zu verwenden ist, zu einer hinreichenden antibakteriellen Wirksamkeit führt. Die Anmelderin hat demgegenüber jedoch in völlig überraschender Weise herausgefunden, dass in der Kombination mit Bisabolol, einem Pflanzenextrakt von gewöhnlicher Moosbeere und einem Pflanzenextrakt von Preiselbeere der Mengenanteil von Mannose in erfindungsgemäßen Zusammensetzungen deutlich gesenkt werden kann, ohne dass eine Abnahme der antibakteriellen Wirksamkeit erfindungsgemäßer Zusammensetzungen beobachtet wird. Hierzu trägt insbesondere bei, dass sich die Komponenten der erfindungsgemäßen Zusammensetzungen in ihren Wirkweise komplementieren und auf synergistische Weise ergänzen, so dass insgesamt die Wirksamkeit einer jeden Komponente selbst über ein erwartbares Maß hinaus gesteigert werden kann, wenn diese, wie im Rahmen der vorliegenden Erfindung vorgesehen, in Kombination miteinander verwendet werden. Entsprechend ist es im Rahmen der vorliegenden Erfindung damit letztlich auch möglich, die Mengenanteile der übrigen Komponenten a) bis c) der erfindungsgemäßen Zusammensetzung vergleichsweise gering zu halten.

Des Weiteren hat es sich im Rahmen der vorliegenden Erfindung im Hinblick auf die übrigen Komponente a) bewährt, wenn die Zusammensetzung, bezogen auf die Gesamtzusammensetzung, die Komponente a) bzw. Bisabolol, insbesondere alpha-Bisabolol, in Mengen im Bereich von 0,01 Gew.-% bis 1,25 Gew.-%, insbesondere in Mengen im Bereich von 0,05 Gew.-% bis 0,75 Gew.-%, vorzugsweise in Mengen im Bereich von 0,075 Gew.-% bis 0,25 Gew.-%, bevorzugt in Mengen im Bereich von 0,085 Gew.-% bis 0,15 Gew.-%, enthält

Gleichmaßen hat es sich für die Komponente b) als geeignet ergeben, wenn die Zusammensetzung, bezogen auf die Gesamtzusammensetzung, die Komponente b) bzw. den Extrakt von Gewöhnlicher Moosbeere (*Vaccinium oxycoccos*) in Mengen im Bereich von 0,1 Gew.-% bis 5 Gew.-%, insbesondere in Mengen im Bereich von 0,25 Gew.-% bis 2,5 Gew.-%, vorzugsweise in Mengen im Bereich von 0,5 Gew.-% bis 1,75 Gew.-%, bevorzugt in Mengen im Bereich von 0,75 Gew.-% bis 1,25 Gew.-%, enthält und/oder

Für die Komponenten c) ist es analog bevorzugt, wenn die Zusammensetzung, bezogen auf die Gesamtzusammensetzung, die Komponente c) bzw. den Extrakt von Preiselbeere (*Vaccinium vitis-idaea*) in Mengen im Bereich von 0,1 Gew.-% bis 5 Gew.-%, insbesondere in Mengen im Bereich von 0,25 Gew.-% bis 2,5 Gew.-%, vorzugsweise in Mengen im Bereich von 0,5 Gew.-% bis 1,75 Gew.-%, bevorzugt in Mengen im Bereich von 0,75 Gew.-% bis 1,25 Gew.-%, enthält.

Wie bereits im Zusammenhang mit der Komponente d) bzw. Mannose ausgeführt, ist es im Rahmen der vorliegenden Erfindung vorteilhaft möglich, vergleichsweise geringe Mengenanteile der Komponenten a) bis c) bzw. insbesondere a) bis d) in der erfindungsgemäßen Zusammensetzung zu verwenden, wobei gleichzeitig vorteilhaft sichergestellt ist, dass hochwirksame bzw. -effiziente Zusammensetzungen erhalten werden, speziell im Hinblick auf eine antibakterielle sowie auch hautpflegende und entzündungslindernde Wirkung der erfindungsgemäßen Zusammensetzung. Im Rahmen der vorliegenden Erfindung wird also vorteilhaft insbesondere eine Zusammensetzung mit einer hohen Wirkeffizienz bei vergleichsweise geringen eingesetzten Mengenanteilen der Zusammensetzungsbestandteile erhalten.

Was nun die Komponente a) weiter im Detail angelangt, so ist es erfindungsgemäß bevorzugt, wenn die Komponente a) bzw. Bisabolol, insbesondere alpha-Bisabolol, synthetisches Bisabolol, insbesondere synthetisches alpha-Bisabolol (Levomenol), ist.

Gleichermaßen hat es sich bewährt, wenn die Komponente a) bzw. Bisabolol, insbesondere alpha-Bisabolol, ein farbloses Öl ist, insbesondere wobei das Öl einen Gehalt an Bisabolol, insbesondere alpha-Bisabolol (Levomenol), im Bereich von mehr als 80 Gew.-%, insbesondere von mehr als 90 Gew.-%, vorzugsweise von mehr als 95 Gew.-%, bevorzugt im Bereich von mehr als 80 Gew.-% bis 99,99 Gew.-%, aufweist.

Alpha-Bisabolol umfasst 4 isomere Verbindungen, wobei erfindungsgemäß insbesondere die Verwendung von (-)-alpha-Bisabolol vorgesehen ist. Die Verwendung von insbesondere synthetischem Bisabolol bzw. alpha-Bisabolol verringert vorteilhaft das eventuelle Risiko bzw. Potential der Bildung bzw. Förderung von Hautunverträglichkeiten bzw. Allergien. Natürliches Bisabolol weist ein relativ hohes allergenes Potenzial auf, welches auf bestimmte, in dem natürlichen Bisabolol enthaltene Pflanzenstoffe zurückführen lässt. Durch die Verwendung von synthetischem Bisabolol, das aufgrund der synthetischen Erzeugung frei von weiteren, insbesondere allergiefördernden Pflanzenstoffen ist, kann der Entstehung bzw. der Förderung von allergischen Reaktionen effektiv entgegengetreten werden und so eine Zusammensetzung enthalten sein, die sich durch eine deutlich gesteigerte Hautverträglichkeit, insbesondere auch in sehr sensiblen Hautarealen bzw. für sehr empfindliche Hautbereiche, auszeichnet.

Durch die vorzugsweise Verwendung von synthetischem Bisabolol ist darüber hinaus die Sicherstellung eines besonders hohen Reinheitsgrades des Bisabolols zuverlässig gegeben. Nicht zuletzt können, indem auf synthetisches Bisabolol zurückgegriffen wird, auch Acker- bzw. Anbauflächen geschont werden, so dass die Verwendung von synthetischem Bisabolol auch eine positive ökologische Komponente mit sich führt.

Im Hinblick auf die Komponente b) bzw. den Extrakt von gewöhnlicher Moosbeere hat es sich im Rahmen der vorliegenden Erfindung bewährt, wenn die Komponente b) bzw. der Extrakt von Gewöhnlicher Moosbeere (*Vaccinium oxycoccos*) ein insbesondere wässriger Flüssigextrakt ist.

Erfindungsgemäß enthält der insbesondere wässrige Flüssigextrakt mindestens einen Trockenextrakt von Gewöhnlicher Moosbeere (*Vaccinium oxycoccos*) und einen Lösevermittler, insbesondere Propylenglykol, bzw. besteht vorzugsweise hieraus.

Dabei enthält der insbesondere wässrige Flüssigextrakt, jeweils bezogen auf die Gesamtzusammensetzung des Flüssigextraktes, vorzugsweise den Trockenextrakt von Gewöhnlicher Moosbeere (*Vaccinium oxycoccos*) in Mengen im Bereich von 0,05 Gew.-% bis 10 Gew.-%, insbesondere in Mengen im Bereich von 0,075 Gew.-% bis 7,5 Gew.-%, vorzugsweise in Mengen im Bereich von 0,085 Gew.-% bis 6,5 Gew.-%, bevorzugt in Mengen im Bereich von 0,09 Gew.-% bis 6 Gew.-%.

Gleichermaßen ist es bevorzugt, wenn der insbesondere wässrige Flüssigextrakt den Lösevermittler in Mengen im Bereich von 10 Gew.-% bis 65 Gew.-%, insbesondere in Mengen im Bereich von 15 Gew.-% bis 60 Gew.-%, vorzugsweise in Mengen im Bereich von 17,5 Gew.-% bis 57,5 Gew.-%, bevorzugt in Mengen im Bereich von 20 Gew.-% bis 55 Gew.-%, enthält.

Die Verwendung von Flüssigextrakten erlaubt vorteilhaft eine effiziente sowie verlässliche Einarbeitbarkeit des Extrakts in die erfindungsgemäße Zusammensetzung, so dass eine homogene, geschmeidige und sensorisch als angenehm empfundene Zusammensetzung enthalten werden kann, die sich insbesondere durch eine kontinuierliche und gleichmäßige Textur auszeichnet. Insbesondere die Verwendung von Propylenglykol als Lösevermittler geht dabei zusätzlich mit dem Vorteil einher, dass sich Propylenglykol durch feuchthaltende, hautpflegende sowie insbesondere auch viskositätsregulierende Eigenschaften auszeichnet, d.h. die Hautpflegeeigenschaften der erfindungsgemäßen Zusammensetzung vorteilhaft ergänzt und unterstützt sowie darüber hinaus auch vorteilhaft zur Formulierung bzw. Einstellung der Viskosität der erfindungsgemäßen Zusammensetzung beitragen kann. Die vorgenannten vorteilhaften Effekte der Extraktbestandteile sind dabei insbesondere in den genannten Mengenbereichen besonders ausgeprägt.

Bevorzugt hat es sich bewährt, wenn der Trockenextrakt zusammen mit und/oder in Kombination mit einem Träger formuliert ist und/oder vorliegt.

Vorzugsweise ist der Träger dabei ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger, bevorzugt Maltodextrin.

Gleichermaßen bevorzugt enthält der Trockenextrakt den Träger in Mengen in einem Bereich von 20 bis 70 Gew.-%, insbesondere 25 bis 60 Gew.-%, vorzugsweise 30 bis 55 Gew.-%, bevorzugt 30 bis 50 Gew.-%, bezogen auf den Trockenextrakt.

Auch ist es im Rahmen der vorliegenden Erfindung vorgesehen, dass der Trockenextrakt von Gewöhnlicher Moosbeere (*Vaccinium oxycoccos*) ein Droge/Extrakt-Verhältnis (DEV) in einem Bereich von 20 : 1 bis 1 : 10, insbesondere 15 : 1 bis 1 : 5, vorzugsweise 10 : 1 bis 1 : 1, bevorzugt 7 : 1 bis 3 : 1, bezogen auf den Trockenextrakt, aufweist.

Das Droge/Extrakt-Verhältnis (DEV) beschreibt hierbei das Verhältnis aus Masse der eingesetzten Droge, d.h. der jeweils eingesetzten Rohstoffmenge der für die eingesetzten Extrakte verwendeten Pflanzen, gegenüber dem gewonnenen, nativen Extrakt ohne weitere Hilfsstoffe bzw. ggf. Träger oder dgl.

Die erfindungsgemäß bevorzugt angestrebten Droge/Extrakt-Verhältnisse garantieren dabei vorteilhaft, dass die mit der erfindungsgemäßen Zusammensetzung assoziierten Vorteile und besonderen technischen Wirkungen, insbesondere die vorteilhaften physiologischen bzw. therapeutischen sowie auch nicht-therapeutischen und kosmetischen Wirkungen, effizient erreicht werden können, insbesondere da hochwirksame bzw. hochkonzentrierte Extrakte im Rahmen der vorliegenden Erfindung bevorzugt eingesetzt werden. Weiterhin gestattet die Verwendung von Pflanzenextrakten, insbesondere von gewöhnlicher Moosbeere, mit dem vorgenannten Droge/Extrakt-Verhältnis eine effiziente Formulierung insbesondere des Flüssigextrakts sowie insgesamt der erfindungsgemäßen Zusammensetzung.

Im Hinblick auf die Komponente c) bzw. den Extrakt von Preiselbeere ist es gleichermaßen wie für die Komponente b) im Rahmen der vorliegenden Erfindung vorgesehen, dass die Komponente c) bzw. der Extrakt von Preiselbeere (*Vaccinium vitis-idaea*) ein insbesondere wässriger Flüssigextrakt ist.

Weiter enthält der insbesondere wässrige Flüssigextrakt mindestens einen Trockenextrakt von Preiselbeere (*Vaccinium vitis-idaea*) und einen Lösevermittler, insbesondere Propylenglykol, bzw. besteht vorzugsweise hieraus.

Es hat sich im Rahmen der vorliegenden Erfindung weiter bewährt, wenn der insbesondere wässrige Flüssigextrakt, jeweils bezogen auf die Gesamtzusammensetzung des Flüssigextraktes, den Trockenextrakt von Preiselbeere (*Vaccinium vitis-idaea*) in Mengen im Bereich von 0,25 Gew.-% bis 7,5 Gew.-%, insbesondere in Mengen im Bereich von 0,5 Gew.-% bis 5 Gew.-%, vorzugsweise in Mengen im Bereich von 0,65 Gew.-% bis 4,5 Gew.-%, bevorzugt in Mengen im Bereich von 0,75 Gew.-% bis 3 Gew.-%, enthält.

Gleichermaßen werden erfindungsgemäß gute Ergebnisse erzielt, wenn der insbesondere wässrige Flüssigextrakt, jeweils bezogen auf die Gesamtzusammensetzung des Flüssigextraktes, den Lösevermittler in Mengen im Bereich von 35 Gew.-% bis 70 Gew.-%, insbesondere in Mengen im Bereich von 40 Gew.-% bis 65 Gew.-%, vorzugsweise in Mengen im Bereich von 45 Gew.-% bis 60 Gew.-%, bevorzugt in Mengen im Bereich von 47,5 Gew.-% bis 55 Gew.-%, enthält.

Indem die Extrakte der Komponente b) bzw. c) im Wesentlichen analog formuliert sind, kann für die Komponente c) zu den diesbezüglichen Vorteilen und besonderen technischen Wirkungen auf den Extrakt von gewöhnlicher Moosbeere bzw. die Komponente b) verwiesen werden und sind diese Vorteile und besonderen technischen Wirkungen entsprechend auch für die Komponente c) bzw. den Extrakt von Preiselbeere zu beobachten.

Ähnlich wie für die Komponenten b) ist es erfindungsgemäß vorzugsweise vorgesehen, dass der Trockenextrakt zusammen mit und/oder in Kombination mit einem Träger formuliert ist und/oder vorliegt.

Insbesondere hat es sich bewährt, wenn der Träger ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger ist, bevorzugt Maltodextrin ist.

Auch hat es sich als vorteilhaft erwiesen, wenn der Trockenextrakt den Träger in Mengen in einem Bereich von 2 bis 10 Gew.-%, insbesondere 3 bis 8 Gew.-%, vorzugsweise 4 bis 6 Gew.-%, bevorzugt 5 Gew.-%, bezogen auf den Trockenextrakt, enthält.

Erfindungsgemäß ist es außerdem vorgesehen, dass der Trockenextrakt von Preiselbeere (*Vaccinium vitis-idaea* L.) ein Droge/Extrakt-Verhältnis (DEV) in einem Bereich von 75 : 1 bis 35 : 1, insbesondere 65 : 1 bis 40 : 1, vorzugsweise 60 : 1 bis 43 : 1, bevorzugt 55 : 1 bis 45 : 1, bezogen auf den Trockenextrakt, aufweist.

Ebenso ist es im Rahmen der Erfindung vorgesehen, dass die Komponente c) bzw. der Extrakt von Preiselbeere (*Vaccinium vitis-idaea* L.) einen Proanthocyanidin-Gehalt in einem Bereich von 0,01 bis 5 Gew.-%, insbesondere 0,05 bis 3,5 Gew.-%, vorzugsweise 0,1 bis 3 Gew.-%, bevorzugt 0,25 bis 2,5 Gew.-%, bezogen auf die Gesamtzusammensetzung des Extrakts, aufweist und/oder wenn der Trockenextrakt von Preiselbeere (*Vaccinium vitis-idaea* L.) einen Proanthocyanidin-Gehalt in einem Bereich von 15 bis 75 Gew.-%, insbesondere 20 bis 65 Gew.-%, vorzugsweise 22,5 bis 55 Gew.-%, bevorzugt 25 bis 50 Gew.-%, bezogen auf den Trockenextrakt, aufweist.

Im Hinblick auf das Droge/Extrakt-Verhältnis des Extrakts von Preiselbeere kann wiederum auf die besonderen Vorteile und technischen Wirkungen, die im Zusammenhang mit der Komponente b) erörtert wurden, verwiesen werden.

Im Hinblick auf den Proanthocyanidin-Gehalt des Extrakts von Preiselbeere bzw. der Komponente c) stellt der Begriff "Proanthocyanidin" im Rahmen der vorliegenden Erfindung zunächst generell einen Sammelbegriff für farblose, phenolische, der Gruppe der Tannine zugeordnete, Naturstoffe, die beim Erhitzen mit verdünnten Mineralsäuren eine Farbreaktion ergeben ("Proanthocyanidin-Reaktion") dar. Die Farbreaktion resultiert aus der Bildung von Anthocyanidin, den farbgebenden Komponenten der Anthocyane, die die rote, violette oder blaue Färbung von Blüten, Blättern und Früchten entsprechender Pflanzen bedingen.

Erfindungsgemäß werden dabei bevorzugt Preiselbeerextrakte mit vergleichsweise hohen Proanthocyanidingehalten in der erfindungsgemäßen Zusammensetzung eingesetzt. Dies gestattet im Rahmen der vorliegenden Erfindung vorteilhaft, dass, wie bereits eingangs ausgeführt, vergleichsweise geringe Extraktmengen in erfindungsgemäßen Zusammensetzungen eingesetzt werden können, ohne dass eine Verringerung der Wirksamkeit bzw. Effektivität der Wirkung der Zusammensetzung beobachtet wird. Insofern kann auf Basis der erfindungsgemäß eingesetzten Pflanzenextrakte effizient und ressourcensparend eine Zusammensetzung formuliert werden, die im Zusammenhang mit der therapeutischen wie auch kosmetischen Verwendung zur Intimpflege bzw. bei der Behandlung von Infektionen des Urogenitaltrakts eine hohe Wirksamkeit bzw. eine besonderen Pflegeeffekt aufweist.

Was nun die weitere Zusammensetzung bzw. die Auswahl weiterer Komponenten für die erfindungsgemäße Zusammensetzung anbelangt, so kann diese im Allgemeinen vielfältig sein. Im Rahmen der vorliegenden Erfindung hat es sich jedoch bewährt, wenn die Zusammensetzung außerdem als Komponente e) Pentylenglykol umfasst.

Insbesondere ist es vorgesehen, dass die Zusammensetzung die Komponente e) bzw. das Pentylenglykol in wirksamen, insbesondere in pharmazeutisch und/oder kosmetisch wirksamen, Mengen enthält und/oder, dass die Zusammensetzung, bezogen auf die Gesamtzusammensetzung, die Komponente e) bzw. das Pentylenglykol in Mengen im Bereich von 1 Gew.-% bis 15 Gew.-%, insbesondere in Mengen im Bereich von 2 Gew.-% bis 10 Gew.-%, vorzugsweise in Mengen im Bereich von 3 Gew.-% bis 8 Gew.-%, bevorzugt in Mengen im Bereich von 3,5 Gew.-% bis 7 Gew.-%, enthält.

Die Verwendung von Pentylenglykol in der erfindungsgemäßen Zusammensetzung trägt vorteilhaft dazu bei, dass die Zusammensetzung im Hinblick auf die Hautpflegeeigenschaften weiter ausgeprägte feuchtigkeitsspendende Eigenschaften aufweist. Insbesondere für empfindliche Hautareale bzw. sehr sensible sowie zur Trockenheit neigende Haut können mit der vorliegenden Erfindung Zusammensetzungen bereitgestellt werden, die sich durch als vorteilhaft zu bewertende Anwendungseigenschaften auszeichnen und die Haut effektiv stärken und gesunderhalten.

Darüber hinaus weist Pentylenglykol auch antimikrobielle Eigenschaften auf, was die antibakteriellen Eigenschaften der Komponenten a) bis d) nochmals vorteilhaft komplementiert. Außerdem wirkt die Komponente e) bzw. Pentylenglykol auch in gewisser Weise konservierend, was die Lagerfähigkeit bzw. Stabilität der erfindungsgemäßen Zusammensetzung vorteilhaft steigert. Die vorgenannten besonderen technischen Wirkungen und Eigenschaften der Komponente e) sind dabei in den erfindungsgemäß bevorzugten Mengenbereichen besonders ausgeprägt und treten dann in der erfindungsgemäßen Zusammensetzung besonders effektiv hervor.

Des Weiteren hat es sich erfindungsgemäß im Hinblick auf weitere mögliche Komponenten bzw. Inhaltsstoffe der erfindungsgemäßen Zusammensetzung als vorteilhaft erwiesen, wenn die Zusammensetzung außerdem als Komponente f) Hyaluronan und/oder physiologisch verträgliche Salze davon, insbesondere physiologisch verträgliche Salze von Hyaluronan, vorzugsweise Natriumhyaluronat, umfasst.

insbesondere wobei die Zusammensetzung die Komponente f) bzw. das Hyaluronan und/oder physiologisch verträgliche Salze davon, insbesondere physiologisch verträgliche Salze von Hyaluronan, vorzugsweise Natrium¬hyaluronat in wirksamen, insbesondere in pharmazeutisch und/oder kosmetisch wirksamen, Mengen enthält; und/oder

Vorzugsweise ist dabei vorgesehen, dass die Zusammensetzung, bezogen auf die Gesamt-zusammensetzung, die Komponente f) bzw. das Hyaluronan und/oder physiologisch verträgliche Salze davon, insbesondere physiologisch verträgliche Salze von Hyaluronan, vorzugsweise Natriumhyaluronat in Mengen im Bereich von 0,05 Gew. % bis 5 Gew. %, insbesondere in Mengen im Bereich von 0,1 Gew. % bis 2,5 Gew. %, vorzugsweise in Mengen im Bereich von 0,25 Gew. % bis 1,5 Gew. %, bevorzugt in Mengen im Bereich von 0,3 Gew. % bis 1 Gew. %, enthält.

Die Komponente f) bzw. Hyaluronan bzw. insbesondere Natriumhyaluronat zeichnet sich vorteilhaft dadurch aus, dass die Komponente feuchthaltende Eigenschaften aufweist und insbesondere auch gestattet, den Wassergehalt eines Kosmetikums bzw. einer Zusammensetzung im Allgemeinen sowohl während der Lagerung als auch beim Auftrag bzw. der Applikation auf die Haut konstant zu erhalten.

Gleichermaßen trägt die Komponente f) dazu bei, den Feuchtigkeitsgehalt der Haut zu steigern und kann so bewirken bzw. erreichen, dass die Haut weich, geschmeidig und elastisch erhalten bleibt. Insbesondere kann die Komponente f) bzw. Hyaluronan bzw. vorzugsweise Natriumhyaluronat dazu beitragen, dass Austrocknen von Schleimhäuten, insbesondere des Urogenitaltraktes, zu verhindern oder aber zumindest abzumildern. Auf diese Weise wird es vorteilhaft möglich, die Haut des Intimbereichs bzw. des Urothel einerseits gesund und gepflegt zu erhalten sowie andererseits die Widerstandsfähigkeit gegenüber Infektionen mit Krankheitserregern zu steigern.

Weiterhin hat es sich erfindungsgemäß bewährt, wenn die Zusammensetzung außerdem als Komponente g) Milchsäure und/oder physiologisch verträglichen Salze davon, insbesondere Milchsäure, umfasst.

Hierbei hat es sich insbesondere bewährt, wenn die Zusammensetzung die Komponente g) bzw. die Milchsäure und/oder physiologisch verträglichen Salze davon, insbesondere Milchsäure in wirksamen, insbesondere in pharmazeutisch und/oder kosmetisch wirksamen, Mengen enthält bzw., wenn die Zusammensetzung, bezogen auf die Gesamtzusammensetzung, die Komponente g) bzw. die Milchsäure und/oder physiologisch verträglichen Salze davon, insbesondere Milchsäure, in Mengen im Bereich von 0,05 Gew.-% bis 5 Gew.-%, insbesondere in Mengen im Bereich von 0,1 Gew.-% bis 2,5 Gew.-%, vorzugsweise in Mengen im Bereich von 0,25 Gew.-% bis 1,5 Gew.-%, bevorzugt in Mengen im Bereich von 0,3 Gew.-% bis 1 Gew.-%, enthält.

Die Komponente g) bzw. Milchsäure dient vorteilhaft zur Einstellung eines physiologischen pH-Wertes in der erfindungsgemäßen Zusammensetzung und gestattet darüber hinaus vorteilhaft auch eine Regulierung des pH-Wertes der Haut, insbesondere des Intimbereichs, im Rahmen der topischen Applikation der erfindungsgemäßen Zusammensetzung. Auf diese Weise kann die Verwendung von Milchsäure in der erfindungsgemäßen Zusammensetzung vorteilhaft zur Kontrolle der bakteriellen Besiedlung der Haut beitragen und diese auf insbesondere gesunde Weise regulieren, so dass die Vermehrung bzw. Verbreitung von Krankheitserregern, wie insbesondere *E. coli,* kontrolliert bzw. minimiert werden kann.

Des Weiteren zeichnet sich Milchsäure dadurch aus, dass sie eine Art hygroskopischen Film auf der Hautoberfläche bilden kann, was zur Feuchthaltung der oberen Hautschichten vorteilhaft beiträgt. Auf Basis der vorgenannten Eigenschaften der Komponente g) bzw. von Milchsäure wird die Wirksamkeit bzw. Effektivität der erfindungsgemäßen Zusammensetzung vorteilhaft und positiv beeinflusst bzw. insbesondere gesteigert. Die erfindungsgemäße Zusammensetzung erhält vorteilhaft einen feuchtigkeitsspendenden Charakter und zeichnet sich durch besonders hautpflegende und -regulierende Eigenschaften aus. Die antibakteriellen Wirksamkeit der erfindungsgemäßen Zusammensetzung wird auf Basis der Komponente g) vorteilhaft komplimentiert, indem die Komponente g) zur pH-Wert-Regulierung der Hautoberfläche beiträgt. Im Zusammenspiel mit den Komponenten a) bis d) der erfindungsgemäßen Zusammensetzung wird so nochmals eine Steigerung der antibakteriellen Eigenschaften der erfindungsgemäßen Zusammensetzung erreicht sowie insbesondere auch eine Steigerung der hautpflegenden kosmetischen Wirkung der erfindungsgemäßen Zusammensetzung.

Die vorteilhaften Wirkungen und besonderen technischen Effekte, die mit dem Zusatz der Komponente g) bzw. von Milchsäure zu der erfindungsgemäßen Zusammensetzung einhergehen, sind insbesondere in den genannten Mengenbereichen besonders vorteilhaft ausgeprägt, so dass innerhalb dieser besonders im Rahmen der vorliegenden Erfindung gute Ergebnisse erzielt werden.

Überdies hat es sich im Rahmen der vorliegenden Erfindung bewährt, wenn die Zusammensetzung außerdem als Komponente h) ein physiologisch verträgliches Antioxidans, Fungizid und/oder Bakterizid, insbesondere aus der Gruppe der Acetophenone, vorzugsweise aus der Gruppe der Hydroxyacetophenone, bevorzugt 4-Hydroxyacetophenon, umfasst.

Gute Ergebnisse werden dabei insbesondere erhalten, wenn die Zusammensetzung die Komponente h) bzw. das physiologisch verträgliche Antioxidans, Fungizid und/oder Bakterizid, insbesondere aus der Gruppe der Acetophenone, vorzugsweise aus der Gruppe der Hydroxyacetophenone, bevorzugt 4-Hydroxyacetophenon, in wirksamen, insbesondere in pharmazeutisch und/oder kosmetisch wirksamen, Mengen enthält bzw., wenn die Zusammensetzung, bezogen auf die Gesamtzusammensetzung, die Komponente h) bzw. das physiologisch verträgliche Antioxidans, Fungizid und/oder Bakterizid, insbesondere aus der Gruppe der Acetophenone, vorzugsweise aus der Gruppe der Hydroxyacetophenone, bevorzugt 4-Hydroxyacetophenon, in Mengen im Bereich von 0,05 Gew.-% bis 5 Gew.-%, insbesondere in Mengen im Bereich von 0,1 Gew.-% bis 2,5 Gew.-%, vorzugsweise in Mengen im Bereich von 0,25 Gew.-% bis 1,5 Gew.-%, bevorzugt in Mengen im Bereich von 0,3 Gew.-% bis 1 Gew.-%, enthält.

Wenn die erfindungsgemäße Zusammensetzung die Komponente h) bzw. ein physiologisch verträgliches Antioxidans, insbesondere 4-Hydroxyacetophenon, enthält, kann vorteilhaft eine weitere Steigerung der antibakteriellen sowie hautpflegenden und -schützenden Eigenschaften der erfindungsgemäßen Zusammensetzung beobachtet werden. Insbesondere wird vorteilhaft ein erhöhter Zellschutz erreicht und die Hautstruktur kann gestärkt und gefestigt werden, so dass insbesondere die empfindlichen Areale des Intimbereichs besser gegen Infektionen mit Krankheitserregern wie beispielsweise *E. coli* geschützt sind. Gleichermaßen hat die Komponente h) auch eine stabilisierende Wirkung auf die erfindungsgemäße Zusammensetzung selbst, was sich vorteilhaft in einer erhöhten Lagerstabilität ausdrückt. Die genannten Wirkungen und besonderen technischen Effekte, die mit der Verwendung der Komponente h) in der erfindungsgemäßen Zusammensetzung einhergehen, treten insbesondere in den genannten Mengenbereichen vorteilhaft hervor.

Darüber hinaus hat es sich im Rahmen der vorliegenden Erfindung als vorteilhaft erwiesen, wenn die Zusammensetzung außerdem als Komponente i) ein physiologisch verträgliches Verdickungsmittel, insbesondere aus der Gruppe der Cellulosederivate, vorzugsweise aus der Gruppe der Celluloseether, bevorzugt Cellulosegummi, umfasst.

Hierbei hat es sich vorzugsweise bewährt, wenn die Zusammensetzung die Komponente i) bzw. das physiologisch verträgliche Verdickungsmittel, insbesondere aus der Gruppe der Cellulosederivate, vorzugsweise aus der Gruppe der Celluloseether, bevorzugt Cellulosegummi, in wirksamen, insbesondere in pharmazeutisch und/oder kosmetisch wirksamen, Mengen enthält; bzw., wenn die Zusammensetzung, bezogen auf die Gesamtzusammensetzung, die Komponente i) bzw. das physiologisch verträgliche Verdickungsmittel, insbesondere aus der Gruppe der Cellulosederivate, vorzugsweise aus der Gruppe der Celluloseether, bevorzugt Cellulosegummi, in Mengen im Bereich von 0,25 Gew.-% bis 7,5 Gew.-%, insbesondere in Mengen im Bereich von 0,5 Gew.-% bis 5 Gew.-%, vorzugsweise in Mengen im Bereich von 0,75 Gew.-% bis 3,5 Gew.-%, bevorzugt in Mengen im Bereich von 1 Gew.-% bis 2 Gew.-%, enthält.

Die Komponente i) trägt vorteilhaft dazu bei, die Viskosität der erfindungsgemäßen Zusammensetzung so einzustellen, dass eine sensorisch angenehme sowie als vorteilhaft zu bewertende Zusammensetzung bzw. Formulierung erhalten werden kann. Weiterhin beeinflusst die Komponente i) auch die Anwendungseigenschaften der erfindungsgemäßen Zusammensetzung positiv, indem sie als Filmbildner zur Erzeugung eines kontinuierlichen Films auf der Haut beiträgt und so ein angenehmes Hautgefühl erzeugt. Die Verteilbarkeit der erfindungsgemäßen Zusammensetzung wird hierdurch positiv beeinflusst, was letztlich auch die Applikation einfacher und effizienter gestaltet. Die vorgenannten technischen Wirkungen treten insbesondere in den genannten Mengenbereichen besonders vorteilhaft hervor.

Im Rahmen der vorliegenden Erfindung wird es außerdem bevorzugt, wenn die Zusammensetzung frei ist von Feuchthaltemitteln auf Basis von Zuckeraustauschstoffen, insbesondere auf Basis von Xylitol.

Des Weiteren hat es sich bewährt, wenn die Zusammensetzung einen pH-Wert in einem Bereich von 2 bis 7, insbesondere in einem Bereich von 2,5 bis 6,75, vorzugsweise in einem Bereich von 2,75 bis 6, bevorzugt in einem Bereich von 3 bis 6, aufweist.

Wenn die erfindungsgemäße Zusammensetzung einen pH-Wert im vorgenannten Bereich aufweist, ist vorteilhaft zu beobachten, dass die erfindungsgemäße Zusammensetzung insbesondere pH-Wert-regulierend auf die Haut, insbesondere des Intimbereichs, einwirken kann, was förderlich dazu beiträgt, die bakterielle Besiedlung der Haut zu regulieren und in einem gesunden Maße zu erhalten. Weiterhin kann ein pH-Wert der erfindungsgemäßen Zusammensetzung im vorgenannten Bereich vorteilhaft zur Stärkung des hauteigenen Säureschutzmantels beitragen, indem dieser komplettiert oder reguliert wird. Die Stärkung des hauteigenen Säureschutzmantels trägt dabei ebenfalls vorteilhaft dazu bei, das Risiko für Infektionen zu verringern, insbesondere da die Widerstandsfähigkeit der Haut gesteigert ist.

Vorteilhaft ist die erfindungsgemäße Zusammensetzung zudem als Creme, Lotion, Cremegel und/oder Gel, insbesondere als Gel ausgebildet.

Es hat sich im Rahmen der vorliegenden Erfindung als vorteilhaft erwiesen, wenn die erfindungsgemäße Zusammensetzung wie zuvor beschrieben formuliert ist, insbesondere, da für entsprechend formulierte Zusammensetzungen besonders positive Anwendungseigenschaften beobachtet werden. Die Sensorik bzw. das Hautgefühl bei Applikation der erfindungsgemäßen Zusammensetzung kann für eine Formulierung wie zuvor genannt als vorteilhaft beschrieben werden, wobei insbesondere der feuchtigkeitsspendende bzw. feuchthaltende Charakter der erfindungsgemäßen Zusammensetzung, insbesondere wenn diese als Gel ausgebildet ist, positiv hervorzuheben ist.

Dies trifft insbesondere auch zu, wenn die erfindungsgemäße Zusammensetzung bei der therapeutischen Behandlung von Infektionen des Urogenitaltraktes angewendet wird, insbesondere da die feuchthaltenden Eigenschaften gleichermaßen auch als Symptom-lindernd bzw. -mildernd empfunden werden und eine Reizung des befallenen Gewebes bzw. der umliegenden Hautareale effizient und nachweislich gesenkt werden können.

In diesem Zusammenhang ist es im Rahmen der vorliegenden Erfindung entsprechend weiter bevorzugt, wenn die Zusammensetzung mindestens einen Gelbildner umfasst.

Vorzugsweise ist der Gelbildner dabei ausgewählt aus der Komponente f) bzw. dem Hyaluronan und/oder physiologisch verträglichen Salze davon, insbesondere physiologisch verträglichen Salze von Hyaluronan, vorzugsweise Natriumhyaluronat, und/oder der Komponente i) bzw. dem physiologisch verträglichen Verdickungsmittel, insbesondere aus der Gruppe der Cellulosederivate, vorzugsweise aus der Gruppe der Celluloseether, bevorzugt dem Cellulosegummi.

Darüber hinaus hat es sich im Rahmen der vorliegenden Erfindung bewährt, wenn die Inhaltsstoffe der Zusammensetzung mit einem physiologisch verträglichen, insbesondere kosmetisch und/oder pharmazeutisch kompatiblen, Exzipienten (Träger) formuliert sind.

Bevorzugt ist der Exzipient (Träger) eine wässrige Lösung, vorzugsweise Wasser.

Es hat sich diesbezüglich bewährt, wenn die Zusammensetzung den Exzipienten (Träger), bezogen auf die Gesamtzusammensetzung, in Mengen im Bereich von 60 Gew.-% bis 99 Gew.-%, insbesondere in Mengen im Bereich von 70 Gew.-% bis 97,5 Gew.-%, vorzugsweise in Mengen im Bereich von 75 Gew.-% bis 95 Gew.-%, bevorzugt in Mengen im Bereich von 80 Gew.-% bis 92,5 Gew.-%, enthält.

Indem der Exzipient im Rahmen der vorliegenden Erfindung bevorzugt eine wässrige Lösung, besonders bevorzugt Wasser ist, wird auch hierdurch der feuchthaltende bzw. feuchtigkeitsspendende Charakter der erfindungsgemäßen Zusammensetzung nochmals zusätzlich unterstützt. Dies trägt vorteilhaft zu einer angenehmen Sensorik und einem angenehmen sowie insbesondere erfrischenden Hautgefühl beim Auftrag der erfindungsgemäßen Zusammensetzung, insbesondere im Intimbereich, bei. Die Haut fühlt sich gepflegt und im Fall akuter Infektionen angenehm gekühlt an, so dass eine lindernde bzw. Symptom-mildernde Sensation verspürt werden kann.

Weiterhin kann es vorgesehen sein, dass die Zusammensetzung mindestens einen (weiteren) Hilfsstoff (Additiv) umfasst;
insbesondere wobei der (weitere) Hilfsstoff (Additiv) ausgewählt ist aus der Gruppe von Füll-, Streck-, Binde-, Netz-, Stabilisierungs-, Färbe-, Puffer-, Riech-, Aroma-, Nähr-, Gleit-, Verarbeitungshilfs- und/oder Konservierungsstoffen und -mitteln sowie deren Kombinationen.

Für weitergehende Einzelheiten zu dem vorstehend beschriebenen Erfindungsaspekt kann zudem Bezug genommen werden auf die Ausführungen zu den weiteren Erfindungsaspekten, wobei diese Ausführungen gleichermaßen für den vorliegenden Erfindungsaspekt entsprechend gelten.

Zudem Gegenstand der vorliegenden Erfindung - gemäß einem **zweiten** Aspekt der vorliegenden Erfindung - ist eine Zusammensetzung, insbesondere pharmazeutische und/oder kosmetische Zusammensetzung, wie zuvor beschrieben zur Verwendung bei der prophylaktischen und/oder therapeutischen Behandlung von Infektionen und/oder entzündlichen Erkrankungen der Harnwege;
zur topischen Applikation im äußeren Intimbereich, insbesondere im äußeren Vaginalbereich, vorzugsweise im Bereich des Harnröhrenausgangs, bevorzugt im Bereich des weiblichen Harnröhrenausgangs, und/oder im Bereich des Dammes, vorzugsweise im Bereich des weiblichen Dammes.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Vorliegend beschrieben ist auch die Verwendung einer Zusammensetzung, wie zuvor beschrieben, zur nicht-therapeutischen und/oder nicht-medizinischen und/oder kosmetischen Vorbeugung von Infektionen und/oder entzündlichen Erkrankungen des Urogenitaltraktes, insbesondere der Harnwege, und/oder zur unterstützenden, vorzugsweise präventiven und/oder kurativen, kosmetischen Verwendung bei der Intimpflege;
insbesondere wobei die Zusammensetzung für die topische Applikation im vorzugsweise äußeren Intimbereich, insbesondere im äußeren Vaginalbereich, vorzugsweise im Bereich des Harnröhrenausgangs, bevorzugt im Bereich des weiblichen Harnröhrenausgangs, und/oder im Bereich des Dammes, vorzugsweise im Bereich des weiblichen Dammes, geeignet und/oder vorgesehen ist und/oder eingesetzt und/oder verwendet wird.

Für weitergehende Einzelheiten zu diesem Aspekt, insbesondere auch zu den assoziierten Vorteilen und besonderen technischen Wirkungen bzw. Effekten, kann Bezug genommen werden auf die Ausführungen zu den Erfindungsaspekten, wobei diese Ausführungen gleichermaßen für den vorliegenden Aspekt entsprechend gelten.

Weiterer Gegenstand der vorliegenden Erfindung - gemäß einem **dritten** Aspekt der vorliegenden Erfindung - ist eine pharmazeutische Zubereitung, insbesondere Creme, Lotion, Cremegel und/oder Gel, vorzugsweise Gel, bevorzugt Arzneimittel oder Medizinprodukt, umfassend eine Zusammensetzung nach der vorliegenden Erfindung, insbesondere zur Verwendung bei der prophylaktischen und/oder therapeutischen Behandlung von Infektionen und/oder entzündlichen Erkrankungen des Urogenitaltraktes, insbesondere der Harnwege;
zur topischen Applikation im äußeren Intimbereich, insbesondere im äußeren Vaginalbereich, vorzugsweise im Bereich des Harnröhrenausgangs, bevorzugt im Bereich des weiblichen Harnröhrenausgangs, und/oder im Bereich des Dammes, vorzugsweise im Bereich des weiblichen Dammes.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Wiederum weiterer Gegenstand der vorliegenden Erfindung - gemäß einem **vierten** Aspekt der vorliegenden Erfindung - ist eine kosmetische Zubereitung, insbesondere Creme, Lotion, Cremegel und/oder Gel, vorzugsweise Gel, umfassend eine Zusammensetzung nach der vorliegenden Erfindung, insbesondere zur nicht-therapeutischen und/oder nicht-medizinischen und/oder kosmetischen Vorbeugung von Infektionen und/oder entzündlichen Erkrankungen des Urogenitaltraktes, insbesondere der Harnwege, und/oder zur unterstützenden, vorzugsweise präventiven und/oder kurativen, kosmetischen Verwendung bei der Intimpflege,
zur topischen Applikation im äußeren Intimbereich, insbesondere im äußeren Vaginalbereich, vorzugsweise im Bereich des Harnröhrenausgangs, bevorzugt im Bereich des weiblichen Harnröhrenausgangs, und/oder im Bereich des Dammes, vorzugsweise im Bereich des weiblichen Dammes.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Zudem weiterer Gegenstand der vorliegenden Erfindung - gemäß einem **fünften** Aspekt der vorliegenden Erfindung - ist die Verwendung einer kosmetischen Zubereitung nach der vorliegenden Erfindung im Intimpflegebereich, insbesondere zur nicht-therapeutischen und/oder nicht-medizinischen und/oder kosmetischen Vorbeugung von Infektionen und/oder entzündlichen Erkrankungen des Urogenitaltraktes, insbesondere der Harnwege, und/oder zur unterstützenden, vorzugsweise präventiven und/oder kurativen, kosmetischen Verwendung bei der Intimpflege,
zur topischen Applikation im äußeren Intimbereich, insbesondere im äußeren Vaginalbereich, vorzugsweise im Bereich des Harnröhrenausgangs, bevorzugt im Bereich des weiblichen Harnröhrenausgangs, und/oder im Bereich des Dammes, vorzugsweise im Bereich des weiblichen Dammes.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Schließlich Gegenstand der vorliegenden Erfindung - gemäß einem **sechsten** Aspekt der vorliegenden Erfindung - ist eine Dosiervorrichtung, vorzugsweise Dosierspender, insbesondere zur bevorzugt portionsweisen Abgabe oder Ausbringung, enthaltend eine Zusammensetzung oder eine Zubereitung nach der vorliegenden Erfindung.

Vorteilhaft gestattet die Bereitstellung der erfindungsgemäßen Zusammensetzung in einer Dosiervorrichtung eine mengengenaue, individuell anpassbare und unkomplizierte Abgabe dieser. Weiterhin vorteilhaft kommt die erfindungsgemäße Zusammensetzung während der Ausbringung bzw. Abgabe aus der Dosiervorrichtung kaum mit Umwelteinflüssen in Kontakt, insbesondere im Hinblick auf den in der Dosiervorrichtung verbleibenden Teil der Zusammensetzung. Auf diese Weise kann eine Kontamination der Zusammensetzung sicher vermieden und die Haltbarkeit der Zusammensetzung über einen langen Zeitraum verlässlich gewährleistet werden.

Erfindungsgemäß hat es sich dabei bewährt, wenn die Dosiervorrichtung als Pumpspender, insbesondere Druckspender ausgebildet ist, wobei die Zusammensetzung oder Zubereitung bei der Abgabe oder Ausbringung nicht mit Umgebungsluft in Kontakt gebracht wird und/oder wobei die Zusammensetzung oder Zubereitung in einer von Umgebungsluft geschützten Umhausung oder Umhüllung eingebracht ist, insbesondere zum Schutz gegenüber Oxidation.

Besonders vorteilhaft ist die Dosiervorrichtung dabei als Airless-Dosierspender, insbesondere Airless-Pumpspender, vorzugsweise Airless-Druckspender, ausgebildet.

Airless-Dosierspender, synonym auch als Airless-Dispenser bezeichnet, zeichnen sich gegenüber herkömmlichen Dispensern durch eine besondere Bauweise aus.

Bei herkömmlichen Dispensern (Dosierspendern), die das Füllgut mittels einer gewöhnlichen Lotionspumpe abgeben bzw. ausbringen, entsteht während der Abgabe dieses, durch das Drücken der Pumpe, welche daraufhin durch eine Feder wieder nach oben gezogen wird, ein Vakuum. Infolgedessen wird das Füllgut im Dispenser über einen mit dem Pumpkopf verbundenen Schlauch nach oben gezogen. Damit der Dispenser durch das Vakuum nicht zusammengezogen wird, werden beim Pumpen Einrichtungen geöffnet, die das Einströmen von Luft in den Dispenser ermöglichen.

Im Gegensatz dazu kann in den Airless-Dispenser auf Grund dessen spezieller Bauweise keine Luft eindringen und so mit dem Füllgut in Kontakt kommen. Ein Airless Dispenser besteht gewöhnlicherweise aus einem Pumpkopf, dem Dispenser-Behälter und einem Kolben. Es gibt hingegen keinen Schlauch, der das Füllgut nach oben saugen und Luft im verbleibenden Füllgut hinterlassen kann. Vielmehr drückt bei einer Ausführung des Airless-Dispensers die durch den Pumpvorgang eintretende Luft über einen Balg im Pumpkopf den sich initial ganz unten im Behälter befindenden Kolben durch Unterdruck nach oben. Dieser Kolben befördert dann das Füllgut mit jedem Pumpstoß immer weiter nach oben, ohne dass austretende Luft in den Dispenser-Behälter einströmen kann. Eine beispielhafte Ausführung eines solchen Airless-Dispensers zeigt u.a. die europäische Patentanmeldung EP 2 017 011 A1.

Vorteilhaft wird mittels des Airless-Dispensers also ein Inkontaktbringen der erfindungsgemäßen Zusammensetzung insbesondere mit Luftsauerstoff vermieden. So kann evtl. degradierenden Oxidationsreaktionen im Dispenser wirkungsvoll vorgebeugt und die Haltbarkeit und Lagerbeständigkeit der erfindungsgemäßen Zusammensetzung vorteilhaft deutlich gesteigert werden. Gleichzeitig ist auch eine Kontamination der Zusammensetzung mit anderen Umweltchemikalien vergleichsweise sicher unterbunden, so dass ein anwendungssicheres und hygienisch unbedenkliches Produkt auch nach einiger Standzeit noch ohne Bedenken bereitgestellt ist bzw. verwendet werden kann.

Die Verwendung von Airless-Dispensern im Rahmen der vorliegenden Erfindung geht also vorteilhaft damit einher, dass Oxidation und Verderb der erfindungsgemäßen Zusammensetzung vorgebeugt wird. Durch das luftdichte System werden die Inhaltsstoffe der Zusammensetzung vor Sauerstoff geschützt, was die Haltbarkeit dieser verlängert und die Wirksamkeit der Produkte auch über einen längeren Zeitraum bewahrt, was vorliegend insbesondere relevant sowie vorteilhaft ist, da die erfindungsgemäße Zusammensetzung teils oxidationsempfindliche Komponenten bzw. Inhaltsstoffe umfasst, deren Wirksamkeit bei Oxidation durch Luftsauerstoff verloren ginge.

Fener verhindert das geschlossene System auch das Eindringen von Keimen, Bakterien oder anderen Verunreinigungen, was eine hygienische Integrität der Zusammensetzung sicherstellt. Dies ist besonders relevant für Produkte, die auf die Haut aufgetragen werden, da so Infektionen oder Irritationen effektiv vermieden werden können. Indem der Airless-Dispenser einen derartigen Schutz der erfindungsgemäßen Zusammensetzung vor Umwelteinflüssen ermöglicht, kann außerdem vorteilhaft auch auf den Einsatz von Konservierungsmitteln im Rahmen der vorliegenden Erfindung verzichtet werden.

Vorteilhaft ist außerdem die Möglichkeit der sehr präzisen und kontrollierten Dosierung der erfindungsgemäßen Zusammensetzung, insbesondere da durch den Druckmechanismus die Menge des abgegebenen Produktes genau reguliert werden kann, wodurch Verschwendung reduziert wird und eine genaue Anwendung gewährleistet ist.

Nicht zuletzt erlaubt die Verwendung von Airless-Dispensern auch ein leichtes Entleeren einer Dosiervorrichtung, denn der Vakuummechanismus sorgt dafür, dass der Inhalt vollständig entleert werden kann, ohne dass Reste im Behälter zurückbleiben. Dies ermöglicht es, dass das Produkt vollständig ausgenutzt wird und Rückstände verringert werden.

Im Rahmen der vorliegenden Erfindung ist es dabei vorzugsweise vorgesehen, dass die Dosiervorrichtung die Zusammensetzung oder Zubereitung in Mengen im Bereich von 5 g bis 200 g, insbesondere im Bereich von 10 g bis 100 g, vorzugsweise im Bereich von 20 g bis 50 g, enthält.

Im Folgenden wird die vorliegende Erfindung anhand von bevorzugten Ausführungsbeispielen näher erläutert. Im Zusammenhang mit der Erläuterung dieser bevorzugten Ausführungsbeispiele bzw. Ausführungsformen der vorliegenden Erfindung, welche jedoch in Bezug auf die vorliegende Erfindung keinesfalls beschränkend sind, werden auch weitergehende Vorteile, Eigenschaften, Aspekte und Merkmale der vorliegenden Erfindung aufgezeigt.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### Ausführungsbeispiele

### 1. Herstellung einer erfindungsgemäßen Zusammensetzung in Form eines Gels

Vorliegend wird eine erfindungsgemäße Zusammensetzung in Form eines Gels auf Basis der nachfolgend in Tabelle 1a angegebenen Grundrezeptur hergestellt.

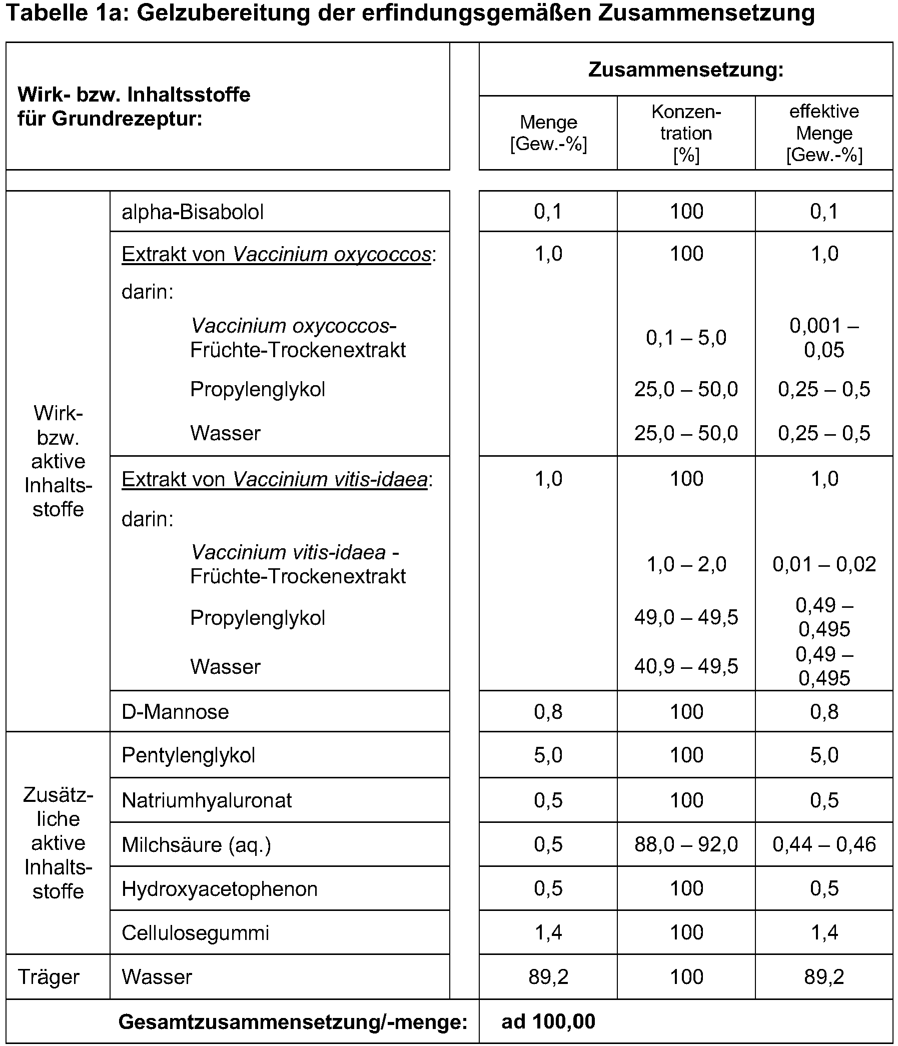

Zudem werden zwei Vergleichszusammensetzungen, welche kein Bisabolol bzw. keine Pflanzenextrakte enthalten, auf Basis der folgenden, in Tabelle 1b bzw. 1c dargestellten Zusammensetzungen hergestellt.

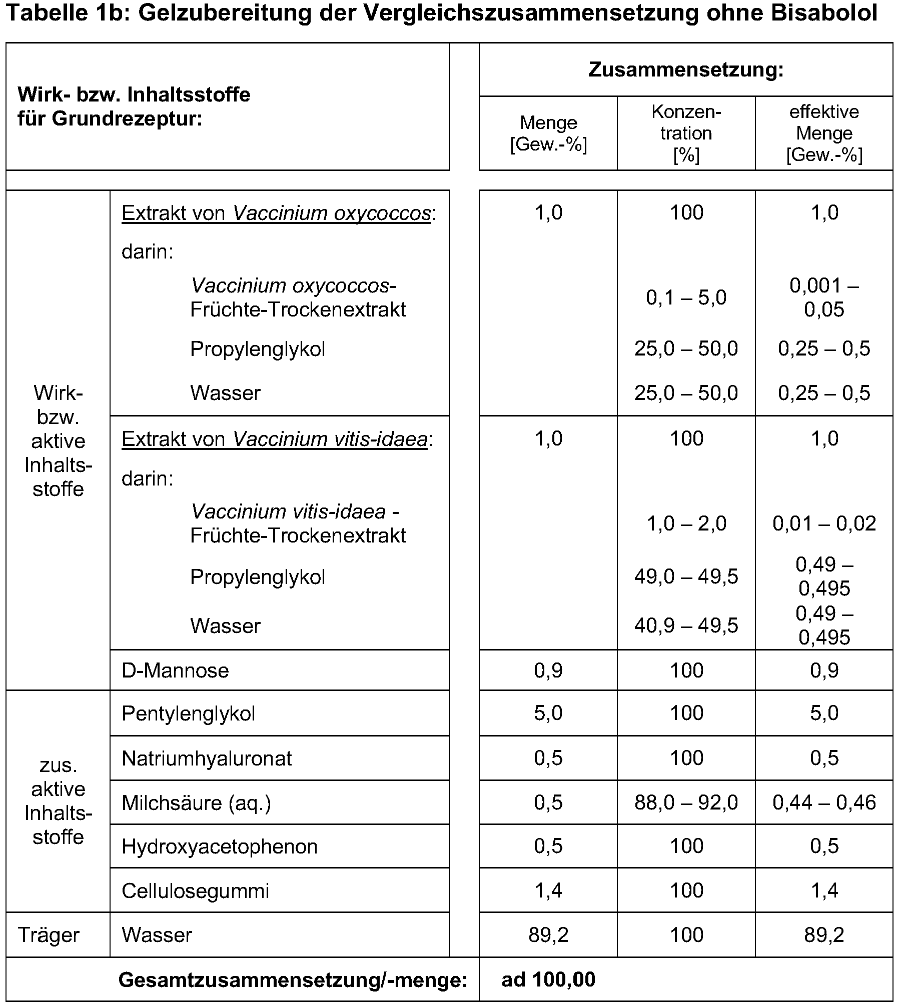

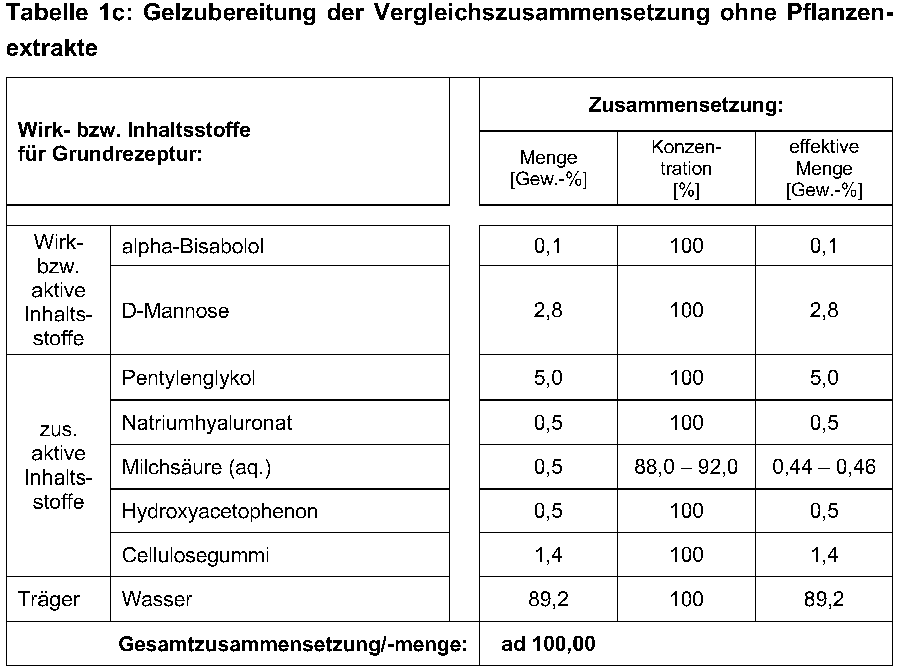

Dabei wird im Allgemeinen derart vorgegangen, dass zunächst alle flüssigen bzw. halb-flüssigen Bestandteile der Zusammensetzung in Wasser eingetragen werden, gefolgt von allen pulverförmigen Bestandteilen. Zuletzt wird schließlich der bzw. die Gelbildner eingetragen und die Mischung unter Einwirkung von Scherkräften zu einer homogenen, viskosen sowie farblosen Gelzusammensetzung vermengt.

Die je in Form eines Gels erhaltenen Zusammensetzungen werden in Mengen von 5 g bis 200 g, insbesondere im Bereich von 10 g bis 100 g, vorzugsweise im Bereich von 20 g bis 50 g abgefüllt, insbesondere jeweils in eine Dosiervorrichtung, die als Pumpspender, insbesondere Druckspender, wie zuvor beschrieben, ausgebildet ist.

Der eingesetzte Extrakt von Preiselbeere *(Vaccinium vitis-idaea* -Früchte-Trockenextrakt) weist ein Droge/Extrakt-Verhältnis (DEV) von ca. 50 : 1 sowie einen Proanthocyanidin-Gehalt von mindestens ca. 25 Gew.-% auf. Der eingesetzte Extrakt von Gewöhnlicher Moosbeere (*Vaccinium* oxycoccos-Früchte-Trockenextrakt) weist ein Droge/Extrakt-Verhältnis (DEV) von ca. 4 - 6 : 1 und einen Proanthocyanidin-Gehalt von mindestens ca. 5 Gew.-% auf.

Die eingesetzten Extrakt von Preiselbeere und von Gewöhnlicher Moosbeere sind jeweils mit Maltodextrin formuliert bzw. geträgert (ca. 5 Gew.-% bzw. ca. 30 bis 50 Gew.-%). Dies ermöglicht eine bessere Verteilbarkeit und Einarbeitbarkeit der Extrakte in die erfindungsgemäße Zusammensetzung, indem eine homogenere und gleichmäßigere Verteilung erreicht wird; es wird zudem auch eine stabilere Formulierung erhalten.

Das eingesetzte alpha-Bisabolol ist synthetisches alpha-Bisabolol und insbesondere unter dem Markennamen Dragosantol^{®} kommerziell erhältlich. Alpha-Bisabolol umfasst vier isomere Verbindungen, wobei vorliegend insbesondere synthetisches (-)-alpha-Bisabolol eingesetzt wird, insbesondere mit einem Reinheitsgrad von über 95 Gew.-%.

D-Mannose wird in pharmazeutisch-kosmetisch einwandfreier Qualität bezogen und als solche direkt in den hergestellten Zusammensetzungen eingesetzt. Gleiches gilt für alle übrigen Inhaltsstoffe der bereitgestellten Zusammensetzungen.

### 2. Untersuchung der Anwendungseigenschaften der Gelzubereitung auf Basis der erfindungsgemäßen Zusammensetzung

### Genereller Studienaufbau:

Zur Analyse der Hautverträglichkeit sowie der Schleimhautverträglichkeit und ggf. des Sensibilisierungspotentials der Gelzubereitung auf Basis der erfindungsgemäßen Zusammensetzung wird eine Anwendungsstudie in Form einer sogenannten Home-In-Use-Testung durchgeführt. Die Gelzubereitung wird dabei im bestimmungsgemäßen Anwendungsareal über einen längeren Zeitraum angewendet, wie nachfolgend beschrieben.

Die Testung erfolgt als monozentrische klinische Prüfung über einen Zeitraum von insgesamt vier Wochen und wird mit 25 weiblichen Probandinnen im Alter zwischen 18 bis 50 Jahren entsprechend den nachfolgenden Ein- und Ausschlusskriterien durchgeführt:
*Einschlusskriterien:*
   - *18 bis 50 Jahre*
   - *weibliche hautgesunde Probanden*
   - *Hauttyp: beliebig*
   - *Probandinnen mit rezidivierender Zystitis*
*Ausschlusskriterien:*
   - *schwere oder chronische Hautentzündungen*
   - *schwere innere oder chronische Erkrankungen*
   - *Einnahme von Medikamenten, die die Hautreaktion beeinträchtigen können (Glucocorticoide, Antiallergika, topische Immunmodulatoren, etc.)*
   - *Applikation von wirkstoffhaltigen Präparaten und Pflegemitteln 7-10 Tage vor Testbeginn*
   - *schwere Allergien oder jemals aufgetretene schwerwiegende Nebenwirkung durch kosmetische Präparate*
   - *Sonnenbäder oder Solariumbesuche während der Studie*
   - *bekanntes Krebsleiden*
   - *Schwangerschaft und Stillzeit*
   - *Nicht intakte Haut oder Schleimhaut im Testareal*

Die Anwendung der erfindungsgemäßen Gelzubereitung erfolgt dreimal täglich im Intimbereich und auf dem rechten Unterarm über den gesamten Anwendungszeitraum. Die Anwendung der Vergleichszubereitungen erfolgt ebenfalls dreimal täglich auf dem rechten Unterarm in einem hinreichend vom ersten Anwendungsbereich beabstandeten zweiten Anwendungsbereich. Die Probandinnen sind angewiesen, während des Testzeitraums kein gleichwertiges Produkt im Testareal zu verwenden.

Zur Evaluation der Anwendungseigenschaften werden mit Hilfe eines abschließenden Fragebogens subjektive, nicht messbare Parameter (subjektiv erlebter Effekt, Geruch, Geschmack, Konsistenz, Einfluss auf das Hautbild, etc.) erfasst. Dazu beantwortet jede Probandin den entsprechenden Fragebogen selbständig zu vorab festgelegten Abfragezeitpunkten. Nachfolgende sind die Untersuchungsergebnisse zu den Anwendungseigenschafften der hergestellten Gelzubereitung zusammengefasst:

### Dermatologische Untersuchungsergebnisse:

Die Untersuchungen sind nach klinisch-dermatologischen Beurteilungskriterien durchgeführt worden. Alle Probandinnen zeigen vor, während und nach der Anwendungsstudie eine gesunde Haut im Bereich der Testareale. Pathologische Hautveränderungen sind in keiner Form festzustellen. Testunterbrechungen oder gar hautfachärztliche Behandlungen sind in keinem Fall durchgeführt worden. Die Gelzubereitung auf Basis der erfindungsgemäßen Zusammensetzung wird sehr gut vertragen und führt bei keiner Probandin zu dermatologisch relevanten Hautveränderungen.

### Gynäkologische Untersuchungsergebnisse:

Die Untersuchungen sind nach klinisch-gynäkologischen Beurteilungskriterien durchgeführt worden. Alle Probandinnen zeigen vor, während und nach der Anwendungsstudie eine gesunde Vaginalschleimhaut. Pathologische Schleimhautveränderungen sind in keiner Form festzustellen. Testunterbrechungen oder gar gynäkologische Behandlungen sind in keinem Fall durchgeführt worden. Die Gelzubereitung auf Basis der erfindungsgemäßen Zusammensetzung wird sehr gut vertragen und führt bei keiner Probandin zu gynäkologisch relevanten Schleimhautveränderungen.

### Auswertung der Beurteilung der Anwendungseigenschaften:

Die Abfrage des subjektiven Eindrucks der Probandinnen zu den Anwendungseigenschaften der Gelzubereitung auf Basis der erfindungsgemäßen Zusammensetzung erfolgte mittels eines abschließenden Fragebogens. Die erhaltenen Ergebnisse sind in der nachfolgenden Tabelle 2 zusammengefasst.

**Tabelle 2: Anwendungseigenschaften der Gelzubereitung auf Basis der erfindungsgemäßen Zusammensetzung**

| **Beurteilungskriterien** | | **Beurteilung [%]** | | | | |
|---|---|---|---|---|---|---|
| | | +++ | ++ | 0 | - | - |
| Gesamtbewertung | | 24 | 64 | 12 | 0 | 0 |
| Hautverträglichkeit | | 72 | 28 | 0 | 0 | 0 |
| Geruchsneutralität | | 76 | 24 | 0 | 0 | 0 |
| sofort verbesserte Hautfeuchtigkeit | | 28 | 56 | 16 | 0 | 0 |
| Positiver Langzeiteffekt auf Hautfeuchte | | 28 | 48 | 24 | 0 | 0 |
| Anwendung bei sensibler Haut | | 36 | 56 | 8 | 0 | 0 |
| Hautpflegeeffekt im Intimbereich | | 44 | 40 | 12 | 4 | 0 |
| Aufbau der natürlichen Hautschutzbarriere | | 8 | 80 | 12 | 0 | 0 |
| Eignung für äußeren Intimbereich | | 56 | 44 | 0 | 0 | 0 |
| Minderung von Hauttrockenheitsgefühl | | 48 | 40 | 8 | 4 | 0 |
| Milderung von Reizungen/Rötungen | | 12 | 40 | 48 | 0 | 0 |
| Schutz vor Trockenheit und Hautirritationen | | 24 | 56 | 20 | 0 | 0 |
| beruhigendes Hautgefühl | | 48 | 44 | 4 | 4 | 0 |
| tägliche Anwendbarkeit | | 60 | 36 | 4 | 0 | 0 |
| verbessertes Intimhygienegefühl | | 32 | 44 | 12 | 12 | 0 |
| Konsistenz | | 84 | 8 | 8 | 0 | 0 |
| einfache/unkomplizierte Handhabung | | 80 | 12 | 4 | 4 | 0 |
| Steigerung des Wohlbefinden | | 40 | 52 | 4 | 4 | 0 |
| Weiterempfehlung | | 96 | 0 | 0 | 4 | 0 |

### Legende:

| sehr gut / voll zustimmend | gut / zustimmend | befriedigend / neutral | ausreichend / nicht zustimmend | mangelhaft / gar nicht zustimmend |
|---|---|---|---|---|
| +++ | ++ | 0 | - | -- |

Insgesamt ist es für die erfindungsgemäße Zusammensetzung bzw. Zubereitung sowie auch die Vergleichszubereitungen aus klinisch-dermatologischer und gynäkologischer Sicht zu keinerlei relevanten Hautreaktionen oder Schleimhautreaktionen im Testareal gekommen und ist die Gelzubereitung auf Basis der erfindungsgemäßen Zusammensetzung sehr gut vertragen worden sowie sehr positiv bewertet worden. Es sind zudem auch weder Intoleranzreaktionen im Sinne einer Irritation noch allergische Reaktionen (Kontaktdermatitis oder Kontaktmukositis) festgestellt worden. Die erfindungsgemäße Zusammensetzung zeichnet sich demnach durch insgesamt als sehr positiv zu bewertende Anwendungseigenschaften aus.

### 3. Untersuchung der Wirksamkeit der Gelzubereitung auf Basis der erfindungsgemäßen Zusammensetzung

Neben der Abfrage der Anwendungseigenschaften wurde im Rahmen der Testung außerdem auch die Wirksamkeit der Gelzubereitung auf Basis der erfindungsgemäßen Zusammensetzung ermittelt. Hierzu wurden die Hautfeuchtigkeit (Corneometrie) sowie der vaginale pH-Wert im Anwendungszeitraum überprüft und evaluiert. Vergleichend werden auch die beiden nichterfindungsgemäßen Gelzubereitungen gemäß den Rezepturen aus Tabelle 1b bzw. 1c im Hinblick auf ihre Wirksamkeit durch Messung der Hautfeuchtigkeit untersucht und wurden die ermittelten Wirksamkeiten einander gegenübergestellt.

### Messung der Hautfeuchtigkeit (Corneometrie):

Bei dem Corneometer-Prinzip wird durch eine Kapazitätsmessung die Hautfeuchtigkeit der "äußeren Schicht", der Oberhaut (Stratum corneum), bestimmt. Die Messung wird mit einem Corneometer CM 825 (Courage + Khazaka electronic GmbH) durchgeführt, dessen Messprinzip auf einer kapazitiven Messung basiert. Der Kondensator des Messkopfes besteht aus zwei voneinander isolierten, parallel angeordneten Platten und misst die Permittivität (ε, dielektrische Leitfähigkeit) eines Dielektrikums (schwach- oder nichtleitende, nichtmetallische Substanz). Wasser besitzt eine hohe relative Permittivität (εr = 80). Je höher also der Wassergehalt der Haut ist, desto höher ist die Permittivität der Haut.

Durch die geringe Eindringtiefe des Corneometers CM 825 (10 bis 20 µm bei Messungen des Stratum corneum) und die Schnelligkeit der Messung (1 Sekunde pro Messung) werden nicht gewollte Einflüsse durch tiefer liegende Strukturen wie z.B. Blutgefäße und eventuell auftretende Okklusionen vermieden. Die Messergebnisse des Corneometer CM 825 werden in relativen Einheiten (arbitrary units = AU, Min. = 0, Max. = 130, Genauigkeit ± 3 %) angegeben. Es werden pro Messzeitpunkt mindestens drei Messungen an verschiedenen Stellen im Testareal durchgeführt. Als Kontrollareal dient ein nicht behandeltes Hautareal.

Es werden für die Gelzubereitung auf Basis der erfindungsgemäßen Zusammensetzung pro Probandin drei Hautfeuchtigkeitsmessungen an verschiedenen Stellen im Testareal (rechter, volarer Unterarm) zu den angegebenen Zeitpunkten durchgeführt und die Werte gemittelt (s. Tabelle 3).

**Tabelle 3: Messung der Hautfeuchtigkeit im Anwendungsareal für die Gelzubereitung auf Basis der erfindungsgemäßen Zusammensetzung gemäß Tabelle 1a**

| **Probandin [Nr.]** | **T₀ [AU]** | **T_{28 Tage} [AU]** | **Differenz [AU]** | **Veränderung [%]** |
|---|---|---|---|---|
| 1 | 39,4 | 43,9 | 4,5 | 11,5 |
| 2 | 35,6 | 43,8 | 8,2 | 23,0 |
| 3 | 41,5 | 46,6 | 5,1 | 12,3 |
| 4 | 29,4 | 52,1 | 22,7 | 77,2 |
| 5 | 37,6 | 53,7 | 16,1 | 42,7 |
| 6 | 36,7 | 47,5 | 10,8 | 29,3 |
| 7 | 38,8 | 47,3 | 8,5 | 21,9 |
| 8 | 55,4 | 69,0 | 13,6 | 24,5 |
| 9 | 38,3 | 60,9 | 22,6 | 59,1 |
| 10 | 34,4 | 57,7 | 23,3 | 67,7 |
| 11 | 36,7 | 46,9 | 10,2 | 27,8 |
| 12 | 38,1 | 52,8 | 14,7 | 38,6 |
| 13 | 32,2 | 44,0 | 11,8 | 36,8 |
| 14 | 32,4 | 46,1 | 13,8 | 42,5 |
| 15 | 28,2 | 49,9 | 21,7 | 76,9 |
| 16 | 59,3 | 64,5 | 5,2 | 8,7 |
| 17 | 30,6 | 41,6 | 11,0 | 35,8 |
| 18 | 41,5 | 54,2 | 12,7 | 30,7 |
| 19 | 47,9 | 51,0 | 3,1 | 6,5 |
| 20 | 31,0 | 53,7 | 22,7 | 73,3 |
| 21 | 33,1 | 38,5 | 5,3 | 16,1 |
| 22 | 38,2 | 43,9 | 5,7 | 14,8 |
| 23 | 51,1 | 70,2 | 19,1 | 37,4 |
| 24 | 42,6 | 52,9 | 10,3 | 24,2 |
| 25 | 36,7 | 46,9 | 10,2 | 27,8 |
| **Durchschnitt** | **38,7** | **51,2** | **12,5** | **34,7** |
| Minimum | 28,2 | 38,5 | 3,1 | 6,5 |
| Maximum | 59,3 | 70,2 | 23,3 | 77,2 |
| Stand.abw. | 7,8 | 8,2 | 6,4 | 21,2 |
| T-Test | p = 8*10⁻¹⁰ | | | |

Wie die in Tabelle 3 referierten Untersuchungen an der erfindungsgemäßen Zusammensetzung gemäß Tabelle 1a zeigen, führt die Anwendung der erfindungsgemäßen Zusammensetzung zu einer signifikanten Veränderung bzw. Verbesserung der Hautfeuchtigkeit im Anwendungsareal (nämlich im Durchschnitt um 34,7 %).

Für die Vergleichszubereitungen gemäß Tabelle 1b und Tabelle 1c werden entsprechende Untersuchungen durchgeführt, welche zwar eine gewisse, aber nur geringfügige Veränderung bzw. Verbesserung der Hautfeuchtigkeit im Anwendungsareal bewirken (nämlich im Durchschnitt nur um 4,2 % für die Vergleichszubereitung gemäß Tabelle 1b und im Durchschnitt nur um 7,1 % für die Vergleichszubereitung gemäß Tabelle 1c).

Schließlich werden außerdem zur Kontrolle drei Hautfeuchtigkeitsmessungen an verschiedenen Stellen im Kontrollareal (linker, volarer Unterarm,) zu den angegebenen Zeitpunkten durchgeführt und die Werte gemittelt (s. Tabelle 4).

**Tabelle 4: Messung der Hautfeuchtigkeit im Kontrollareal**

| **Probandin [Nr.]** | **T₀ [AU]** | **T_{28 Tage} [AU]** | **Differenz [AU]** | **Veränderung [%]** |
|---|---|---|---|---|
| 1 | 41,1 | 43,4 | 2,3 | 5,7 |
| 2 | 33,8 | 35,7 | 1,9 | 5,6 |
| 3 | 42,4 | 36,6 | -5,8 | -13,7 |
| 4 | 29,1 | 41,6 | 12,5 | 43,0 |
| 5 | 36,7 | 47,5 | 10,8 | 29,4 |
| 6 | 43,9 | 43,1 | -0,8 | -1,9 |
| 7 | 41,4 | 40,6 | -0,8 | -1,9 |
| 8 | 50,6 | 52,3 | 1,8 | 3,5 |
| 9 | 40,7 | 43,2 | 2,6 | 6,3 |
| 10 | 39,1 | 42,0 | 2,9 | 7,5 |
| 11 | 44,3 | 34,2 | -10,2 | -22,9 |
| 12 | 34,8 | 42,7 | 7,9 | 22,7 |
| 13 | 42,2 | 42,0 | -0,2 | -0,5 |
| 14 | 41,6 | 40,8 | -0,7 | -1,8 |
| 15 | 49,0 | 50,3 | 1,3 | 2,6 |
| 16 | 52,2 | 52,3 | 0,0 | 0,0 |
| 17 | 38,2 | 39,4 | 1,2 | 3,1 |
| 18 | 44,1 | 42,1 | -2,0 | -4,5 |
| 19 | 41,4 | 39,8 | -1,6 | -3,9 |
| 20 | 38,7 | 39,5 | 0,9 | 2,2 |
| 21 | 40,8 | 41,1 | 0,3 | 0,7 |
| 22 | 36,8 | 36,8 | 0,0 | 0,0 |
| 23 | 62,4 | 62,1 | -0,3 | -0,5 |
| 24 | 54,3 | 53,3 | -1,0 | -1,8 |
| 25 | 35,7 | 34,7 | -1,0 | -2,8 |
| **Durchschnitt** | **42,2** | **43,1** | **0,9** | **3,0** |
| Minimum | 29,1 | 34,2 | -10,2 | -22,9 |
| Maximum | 62,4 | 62,1 | 12,5 | 43,0 |
| Stand.abw. | 7,1 | 6,6 | 4,5 | 12,8 |
| T-Test | p = 0,34 | | | |

Für die erfindungsgemäße Zusammensetzung wird im Anwendungsbereich zum zweiten Messzeitpunkt nach 28-tägiger Anwendung gegenüber dem Ausgangszeitpunkt vor Beginn der Anwendung durchschnittlich eine um 34,7 % gesteigerte Hautfeuchtigkeit gemessen (vgl. Tabelle 3).

Im Kontrollbereich ist die Hautfeuchtigkeit im Wesentlichen unverändert (vgl. Tabelle 4).

Für die Vergleichszusammensetzungen wird eine deutlich geringere Veränderung der Hautfeuchtigkeit festgestellt (nämlich im Durchschnitt nur um 4,2 % für die Vergleichszubereitung gemäß Tabelle 1b und im Durchschnitt nur um 7,1 % für die Vergleichszubereitung gemäß Tabelle 1c), die im Bereich der Kontrollmessung bzw. im Bereich der für den Kontrollbereich ermittelten Werte liegt, so dass für die Vergleichszusammensetzungen allenfalls von einer nur leicht gesteigerten Hautfeuchtigkeit ausgegangen werden kann. Eindeutig liegt der Effekt der Steigerung der Hautfeuchtigkeit für die erfindungsgemäße Zusammensetzung deutlich über den mit den Vergleichszusammensetzungen erzielten Effekten.

Die besonders vorteilhaften Wirkungen der erfindungsgemäßen Zusammensetzung, insbesondere im Hinblick auf die Stärkung der Haut und Förderung und Regulierung der Hautfeuchtigkeit, werden vor diesem Hintergrund also eindrücklich illustriert.

Aus den Untersuchungsergebnissen kann somit abgeleitet werden, dass die Gelzubereitung auf Basis der erfindungsgemäßen Zusammensetzung zu einer deutlichen Steigerung der Hautfeuchtigkeit beiträgt. Eine hohe Hautfeuchtigkeit bzw. Hydratation ist ein Anzeichen einer gesunden Barrierewirkung der Haut, die gegenüber dem Eindringen von bspw. Krankheitserregern eine hohe Resistenz aufweist.

### Messung des vaginalen pH-Wertes:

Die Haut besitzt einen sogenannten natürlichen Säureschutzmantel. Aufgrund der Hautfeuchtigkeit und der natürlich ausgeschiedenen Kohlensäure liegt der pH-Wert der Haut bei ca. 5. Je nach Alter, Geschlecht oder Hautareal variiert der pH-Wert. Das leicht saure Milieu der Hautoberfläche hemmt das Wachstum von pathogenen Mikroorganismen und ist besonders günstig für das Wachstum der Mikroorganismen der Normalflora wie z. B. *Staphylococcus epidermis.* Des Weiteren funktionieren viele pH-abhängige Enzyme, die an dem Aufbau und der Regeneration der Hautbarriere beteiligt sind, optimal bei einem leicht sauren Milieu.

Pro Messzeitpunkt wird im Rahmen der erfolgten Testung eine einmalige, vaginale Messung des pH-Wertes mit Hilfe eines Teststreifens durchgeführt.

Für gesunde Haut unter normalen Raumbedingungen (20°C, Luftfeuchtigkeit 40 bis 60 %) gelten dabei folgende Werte:

| | | | | | | | | | | | |
|---|---|---|---|---|---|---|---|---|---|---|---|
| **pH-Wert** | < 3,5 | 3,8 | 4,0 | 4,3 | 4,5 | 5,5 | 5,7 | 5,9 | 6,2 | 6,5 | >6,5 |
| Frau | + | saurer Bereich | | - | normal | | - | hoher Haut pH-Wert | | | + |
| Mann | + saurer Bereich - | | | normal | | | - | hoher Haut pH-Wert | | | + |

Aufgrund intraindividueller Unterschiede kann ein pH-Wert außerhalb des normalen Bereichs trotzdem einen gesunden Hautzustand bedeuten und ist nicht zwingend krankheitsbedingt.

Die Ergebnisse der vaginalen pH-Wertmessung sind in der nachfolgenden Tabelle 5 wiedergegeben.

**Tabelle 5: Messung des vaginalen pH-Wertes**

| **Probandin [Nr.]** | **T₀** | **T_{28 Tage}** | **Differenz** | **Veränderung [%]** |
|---|---|---|---|---|
| 1 | 4,0 | 4,0 | 0,0 | 0,0 |
| 2 | 4,5 | 4,5 | 0,0 | 0,0 |
| 3 | 4,0 | 4,0 | 0,0 | 0,0 |
| 4 | 4,0 | 4,5 | 0,5 | 12,5 |
| 5 | 4,5 | 4,5 | 0,0 | 0,0 |
| 6 | 4,5 | 4,5 | 0,0 | 0,0 |
| 7 | 4,0 | 4,0 | 0,0 | 0,0 |
| 8 | 6,0 | 4,0 | -2,0 | -33,3 |
| 9 | 3,5 | 4,0 | 0,5 | 14,3 |
| 10 | 4,0 | 4,0 | 0,0 | 0,0 |
| 11 | 4,0 | 4,0 | 0,0 | 0,0 |
| 12 | 4,0 | 4,0 | 0,0 | 0,0 |
| 13 | 4,5 | 4,5 | 0,0 | 0,0 |
| 14 | 4,0 | 4,0 | 0,0 | 0,0 |
| 15 | 4,0 | 4,0 | 0,0 | 0,0 |
| 16 | 4,0 | 4,0 | 0,0 | 0,0 |
| 17 | 4,0 | 4,0 | 0,0 | 0,0 |
| 18 | 4,0 | 4,0 | 0,0 | 0,0 |
| 19 | 4,0 | 4,0 | 0,0 | 0,0 |
| 20 | 4,0 | 4,0 | 0,0 | 0,0 |
| 21 | 4,5 | 4,5 | 0,0 | 0,0 |
| 22 | 4,5 | 4,5 | 0,0 | 0,0 |
| 23 | 4,0 | 6,0 | 2,0 | 50,0 |
| 24 | 4,0 | 4,0 | 0,0 | 0,0 |
| 25 | 4,0 | 4,0 | 0,0 | 0,0 |
| **Durchschnitt** | 4,2 | 4,2 | 0,0 | 1,0 |
| Minimum | 3,5 | 4,0 | -2,0 | -33,3 |
| Maximum | 6,0 | 6,0 | 2,0 | 50,0 |
| Stand.abw. | 0,5 | 0,4 | 0,6 | 12,7 |

Insgesamt kann anhand der Untersuchungsergebnisse ein pH-Wert regulierender Effekt der Gelzubereitung auf Basis der erfindungsgemäßen Zusammensetzung abgeleitet werden. Im Falle hoher Haut-pH-Werte kann mit der erfindungsgemäßen Zusammensetzung eine Absenkung des pH-Wert in einen normalen bzw. sauren pH-Wertbereich erzielt werden, insbesondere wobei ein leicht sauer pH-Wert im Vaginalbereich physiologisch vorherrscht und eine entsprechend pH-Wertabsenkung als positiv zu beurteilen ist. Die Gelzubereitung auf Basis der erfindungsgemäßen Zusammensetzung trägt somit dazu bei, einen gesunden pH-Wert im Vaginalbereich zu erhalten, was insbesondere für die Prophylaxe bzw. bei der Prävention von Harnwegsinfekten sehr vorteilhaft ist und auch im Hinblick auf eine wirkungsvolle Intimpflege, welche zu einer effektiven Gesunderhaltung des Intimbereichs beiträgt, als sehr positiv zu bewerten ist.

Schließlich berichten einzelne Probandinnen auch von einer sofort lindernden Wirkung und einer positiven Beeinflussung des Heilungsverlaufes bei einer akuten Zystitis, so dass die Wirksamkeit der erfindungsgemäßen Zusammensetzung auch insoweit festgestellt werden kann.

Insgesamt zeichnet sich die erfindungsgemäße Zusammensetzung durch als sehr positiv zu bewertende Anwendungseigenschaften aus sowie auch durch eine nachgewiesene vorteilhafte Wirksamkeit insbesondere im Zusammenhang mit der Gesunderhaltung des Intimbereichs, wobei gleichermaßen auch die Wirksamkeit im Rahmen der Anwendung der erfindungsgemäßen Zusammensetzung bei akuter Zystitis nachgewiesen werden kann.

## Patentansprüche

1. Zusammensetzung, insbesondere pharmazeutische und/oder kosmetische Zusammensetzung, insbesondere für die topische Applikation im äußeren Intimbereich, vorzugsweise zur Verwendung bei der prophylaktischen und/oder therapeutischen Behandlung von Infektionen und/oder entzündlichen Erkrankungen des Urogenitaltraktes,
wobei die Zusammensetzung in Kombination und in jeweils wirksamen, insbesondere in jeweils pharmazeutisch und/oder kosmetisch wirksamen, Mengen
a) Bisabolol,
b) mindestens einen Extrakt von Gewöhnlicher Moosbeere (*Vaccinium oxycoccos*), wobei der Extrakt von Gewöhnlicher Moosbeere (*Vaccinium oxycoccos*) ein Flüssigextrakt ist,
wobei der Flüssigextrakt mindestens einen Trockenextrakt von Gewöhnlicher Moosbeere (*Vaccinium oxycoccos)* und einen Lösevermittler enthält,
wobei der Trockenextrakt von Gewöhnlicher Moosbeere (*Vaccinium oxycoccos*) ein Droge/Extrakt-Verhältnis (DEV) in einem Bereich von 20 : 1 bis 1 : 10, bezogen auf den Trockenextrakt, aufweist,
c) mindestens einen Extrakt von Preiselbeere (*Vaccinium vitis-idaea* L*.*),
wobei der Extrakt von Preiselbeere (*Vaccinium vitis-idaea* L.) ein Flüssigextrakt ist, wobei der Flüssigextrakt mindestens einen Trockenextrakt von Preiselbeere (*Vaccinium vitis-idaea* L.) und einen Lösevermittler enthält,
wobei der Trockenextrakt von Preiselbeere (*Vaccinium vitis-idaea* L.) ein Droge/Extrakt-Verhältnis (DEV) in einem Bereich von 75 : 1 bis 35 : 1, bezogen auf den Trockenextrakt, aufweist und
wobei der Trockenextrakt von Preiselbeere (*Vaccinium vitis-idaea* L.) einen Proanthocyanidin-Gehalt in einem Bereich von 0,01 bis 5 Gew.-%, bezogen auf die Gesamtzusammensetzung des Extrakts, aufweist,
d) Mannose,
umfasst;
wobei die Zusammensetzung, bezogen auf die Gesamtzusammensetzung,
die Komponente a) in Mengen im Bereich von 0,01 Gew.-% bis 1,25 Gew.-% enthält,
die Komponente b) in Mengen im Bereich von 0,1 Gew.-% bis 5 Gew.-% enthält,
die Komponente c) in Mengen im Bereich von 0,1 Gew.-% bis 5 Gew.-% enthält,
die Komponente d) in Mengen von weniger als 1 Gew.-% enthält.

2. Zusammensetzung nach Anspruch 1
wobei die Zusammensetzung, bezogen auf die Gesamtzusammensetzung, die Komponente d) in Mengen von weniger als 0,95 Gew.-%, vorzugsweise in Mengen von weniger als 0,9 Gew.-%, bevorzugt in Mengen von weniger als 0,85 Gew.-%, enthält und/oder in Mengen im Bereich von 0,1 Gew.-% bis 0,99 Gew.-%, insbesondere in Mengen im Bereich von 0,2 Gew.-% bis 0,95 Gew.-%, vorzugsweise in Mengen im Bereich von 0,4 Gew.-% bis 0,9 Gew.-%, bevorzugt in Mengen im Bereich von 0,5 Gew.-% bis 0,85 Gew.-%, enthält.

3. Zusammensetzung nach Anspruch 1 oder 2,
wobei die Zusammensetzung, bezogen auf die Gesamtzusammensetzung,
die Komponente a) in Mengen im Bereich von 0,05 Gew.-% bis 0,75 Gew.-%, vorzugsweise in Mengen im Bereich von 0,075 Gew.-% bis 0,25 Gew.-%, bevorzugt in Mengen im Bereich von 0,085 Gew.-% bis 0,15 Gew.-%, enthält und/oder
die Komponente b) in Mengen im Bereich von 0,25 Gew.-% bis 2,5 Gew.-%, vorzugsweise in Mengen im Bereich von 0,5 Gew.-% bis 1,75 Gew.-%, bevorzugt in Mengen im Bereich von 0,75 Gew.-% bis 1,25 Gew.-%, enthält und/oder
die Komponente c) in Mengen im Bereich von 0,25 Gew.-% bis 2,5 Gew.-%, vorzugsweise in Mengen im Bereich von 0,5 Gew.-% bis 1,75 Gew.-%, bevorzugt in Mengen im Bereich von 0,75 Gew.-% bis 1,25 Gew.-%, enthält.

4. Zusammensetzung nach einem der vorangehenden Ansprüche,
wobei der Trockenextrakt von Gewöhnlicher Moosbeere (*Vaccinium oxycoccos*) zusammen mit und/oder in Kombination mit einem Träger formuliert ist und/oder vorliegt;
wobei der Träger ein wasserlöslicher oder wasserdispergierbarer, insbesondere Kohlenhydrat-basierter und/oder -haltiger, vorzugsweise Oligo- und/oder Polysaccharidbasierter und/oder -haltiger, Träger ist, bevorzugt Maltodextrin ist, und/oder
wobei der Trockenextrakt den Träger in Mengen in einem Bereich von 20 bis 70 Gew.-%, insbesondere 25 bis 60 Gew.-%, vorzugsweise 30 bis 55 Gew.-%, bevorzugt 30 bis 50 Gew.-%, bezogen auf den Trockenextrakt, enthält; und/oder
wobei der Trockenextrakt von Gewöhnlicher Moosbeere (*Vaccinium oxycoccos*) ein Droge/Extrakt-Verhältnis (DEV) in einem Bereich von 15 : 1 bis 1 : 5, vorzugsweise 10 : 1 bis 1 : 1, bevorzugt 7 : 1 bis 3 : 1, bezogen auf den Trockenextrakt, aufweist.

5. Zusammensetzung nach einem der vorangehenden Ansprüche,
wobei der Trockenextrakt von Preiselbeere (*Vaccinium vitis-idaea* L.) ein Droge/Extrakt-Verhältnis (DEV) in einem Bereich von 65 : 1 bis 40 : 1, vorzugsweise 60 : 1 bis 43 : 1, bevorzugt 55 : 1 bis 45 : 1, bezogen auf den Trockenextrakt, aufweist und/oder
wobei die Komponente c) bzw. der Extrakt von Preiselbeere (*Vaccinium vitis-idaea* L.) einen Proanthocyanidin-Gehalt in einem Bereich von 0,05 bis 3,5 Gew.-%, vorzugsweise 0,1 bis 3 Gew.-%, bevorzugt 0,25 bis 2,5 Gew.-%, bezogen auf die Gesamtzusammensetzung des Extrakts, aufweist und/oder
wobei der Trockenextrakt von Preiselbeere (*Vaccinium vitis-idaea* L.) einen Proanthocyanidin-Gehalt in einem Bereich von 15 bis 75 Gew.-%, insbesondere 20 bis 65 Gew.-%, vorzugsweise 22,5 bis 55 Gew.-%, bevorzugt 25 bis 50 Gew.-%, bezogen auf den Trockenextrakt, aufweist.

6. Zusammensetzung nach einem der vorangehenden Ansprüche,
wobei die Zusammensetzung außerdem als Komponente e) Pentylenglykol umfasst; und/oder
wobei die Zusammensetzung außerdem als Komponente f) Hyaluronan und/oder physiologisch verträgliche Salze davon, insbesondere physiologisch verträgliche Salze von Hyaluronan, vorzugsweise Natriumhyaluronat, umfasst; und/oder
wobei die Zusammensetzung außerdem als Komponente g) Milchsäure und/oder physiologisch verträglichen Salze davon, insbesondere Milchsäure, umfasst; und/oder
wobei die Zusammensetzung außerdem als Komponente h) ein physiologisch verträgliches Antioxidans, Fungizid und/oder Bakterizid, insbesondere aus der Gruppe der Acetophenone, vorzugsweise aus der Gruppe der Hydroxyacetophenone, bevorzugt 4-Hydroxyacetophenon, umfasst; und/oder
wobei die Zusammensetzung außerdem als Komponente i) ein physiologisch verträgliches Verdickungsmittel, insbesondere aus der Gruppe der Cellulosederivate, vorzugsweise aus der Gruppe der Celluloseether, bevorzugt Cellulosegummi, umfasst.

7. Zusammensetzung nach einem der vorangehenden Ansprüche,
wobei die Zusammensetzung einen pH-Wert in einem Bereich von 2 bis 7, insbesondere in einem Bereich von 2,5 bis 6,75, vorzugsweise in einem Bereich von 2,75 bis 6, bevorzugt in einem Bereich von 3 bis 6, aufweist.

8. Zusammensetzung nach einem der vorangehenden Ansprüche,
wobei die Zusammensetzung als Creme, Lotion, Cremegel und/oder Gel, insbesondere als Gel, ausgebildet ist.

9. Zusammensetzung nach einem der vorangehenden Ansprüche,
wobei die Zusammensetzung mindestens einen Gelbildner umfasst,
insbesondere wobei der Gelbildner ausgewählt ist aus der Komponente f) und/oder der Komponente i).

10. Zusammensetzung, insbesondere pharmazeutische und/oder kosmetische Zusammensetzung, nach einem der vorangehenden Ansprüche zur Verwendung bei der prophylaktischen und/oder therapeutischen Behandlung von Infektionen und/oder entzündlichen Erkrankungen der Harnwege;
zur topischen Applikation im äußeren Intimbereich, insbesondere im äußeren Vaginalbereich, vorzugsweise im Bereich des Harnröhrenausgangs, bevorzugt im Bereich des weiblichen Harnröhrenausgangs, und/oder im Bereich des Dammes, vorzugsweise im Bereich des weiblichen Dammes.

11. Pharmazeutische Zubereitung, insbesondere Creme, Lotion, Cremegel und/oder Gel, vorzugsweise Gel, bevorzugt Arzneimittel oder Medizinprodukt, umfassend eine Zusammensetzung nach einem der Ansprüche 1 bis 9,
zur topischen Applikation im äußeren Intimbereich, insbesondere im äußeren Vaginalbereich, vorzugsweise im Bereich des Harnröhrenausgangs, bevorzugt im Bereich des weiblichen Harnröhrenausgangs, und/oder im Bereich des Dammes, vorzugsweise im Bereich des weiblichen Dammes.

12. Kosmetische Zubereitung, insbesondere Creme, Lotion, Cremegel und/oder Gel, vorzugsweise Gel, umfassend eine Zusammensetzung nach einem der Ansprüche 1 bis 9, zur topischen Applikation im äußeren Intimbereich, insbesondere im äußeren Vaginalbereich, vorzugsweise im Bereich des Harnröhrenausgangs, bevorzugt im Bereich des weiblichen Harnröhrenausgangs, und/oder im Bereich des Dammes, vorzugsweise im Bereich des weiblichen Dammes.

13. Verwendung einer kosmetischen Zubereitung nach Anspruch 12 im Intimpflegebereich zur topischen Applikation im äußeren Intimbereich, insbesondere im äußeren Vaginalbereich, vorzugsweise im Bereich des Harnröhrenausgangs, bevorzugt im Bereich des weiblichen Harnröhrenausgangs, und/oder im Bereich des Dammes, vorzugsweise im Bereich des weiblichen Dammes.

14. Dosiervorrichtung, vorzugsweise Dosierspender, insbesondere zur bevorzugt portionsweisen Abgabe oder Ausbringung, enthaltend eine Zusammensetzung nach einem der Ansprüche 1 bis 9 oder eine Zubereitung nach Anspruch 11 oder 12.

## Claims

1. Composition, in particular pharmaceutical and/or cosmetic composition, in particular for topical application to the external intimate area, preferably for use in the prophylactic and/or therapeutic treatment of infections and/or inflammatory diseases of the urogenital tract,
wherein the composition comprises, in combination and in respective effective, in particular in respective pharmaceutically and/or cosmetically effective, amounts
a) bisabolol,
b) at least one extract of common cranberry (*Vaccinium oxycoccos*),
wherein the common cranberry (*Vaccinium oxycoccos*) extract is a liquid extract, wherein the liquid extract comprises at least one dry extract of common cranberry (*Vaccinium oxycoccos*) and a solubilizing agent,
wherein the dry extract of common cranberry (*Vaccinium oxycoccos*) has a drug/extract ratio (DEV) ranging from 20 : 1 to 1 : 10, based on the dry extract,
c) at least one extract of lingonberry (*Vaccinium vitis-idaea L.*),
wherein the extract of lingonberry (*Vaccinium vitis-idaea* L.) is a liquid extract,
wherein the liquid extract comprises at least one dry extract of lingonberry (*Vaccinium vitis-idaea* L.) and a solubilizing agent,
wherein the dry extract of lingonberry (*Vaccinium vitis-idaea* L.) has a drug/extract ratio (DEV) ranging from 75 : 1 to 35 : 1, based on the dry extract, and
wherein the dry extract of lingonberry (*Vaccinium vitis-idaea* L.) has a proanthocyanidin content ranging from 0.01 to 5 wt.-%, based on the total composition of the extract,
d) mannose;
wherein the composition, based on the total composition,
comprises component a) in amounts ranging from 0.01 wt.-% to 1.25 wt.-%,
comprises component b) in amounts ranging from 0.1 wt.-% to 5 wt.-%,
comprises component c) in amounts ranging from 0.1 wt.-% to 5 wt.-%,
comprises component d) in amounts of less than 1 wt.-%.

2. Composition according to claim 1,
wherein the composition, based on the total composition, comprises component d) in amounts of less than 0.95 wt.-%, preferably in amounts of less than 0.9 wt.-%, more preferably in amounts of less than 0.85 wt.-%, and/or in amounts ranging from 0.1 wt.-% to 0.99 wt.-%, in particular in amounts ranging from 0.2 wt.-% to 0.95 wt.-%, preferably in amounts ranging from 0.4 wt.-% to 0.9 wt.-%, more preferably in amounts ranging from 0.5 wt.-% to 0.85 wt.-%.

3. Composition according to claim 1 or 2,
wherein the composition, based on the total composition,
comprises component a) in amounts ranging from 0.05 wt.-% to 0.75 wt.-%, preferably in amounts ranging from 0.075 wt.-% to 0.25 wt.-%, more preferably in amounts ranging from 0.085 wt.-% to 0.15 wt.-%, and/or
comprises component b) in amounts ranging from 0.25 wt.-% to 2.5 wt.-%, preferably in amounts ranging from 0.5 wt.-% to 1.75 wt.-%, more preferably in amounts ranging from 0.75 wt.-% to 1.25 wt.-%, and/or
comprises component c) in amounts ranging from 0.25 wt.-% to 2.5 w.-%, preferably in amounts ranging from 0.5 wt.-% to 1.75 wt.-%, more preferably in amounts ranging from 0.75 wt.-% to 1.25 wt.-%.

4. Composition according to any one of the preceding claims,
wherein the dry extract of common cranberry (*Vaccinium oxycoccos*) is formulated and/or is present together with and/or in combination with an excipient;
wherein the excipient is a water-soluble or water-dispersible excipient, in particular based on and/or comprising carbohydrates, preferably based on and/or comprising oligo- and/or polysaccharides, preferably wherein the excipient is maltodextrin, and/or
wherein the dry extract comprises the excipient in amounts ranging from 20 to 70 wt.-%, in particular 25 to 60 wt.-%, preferably 30 to 55 wt.-%, more preferably 30 to 50 wt.-%, based on the dry extract; and/or
wherein the dry extract of common cranberry (*Vaccinium oxycoccos*) has a drug/extract ratio (DEV) ranging from 15 : 1 to 1 : 5, preferably 10 : 1 to 1 : 1, more preferably 7 : 1 to 3 : 1, based on the dry extract.

5. Composition according to any one of the preceding claims,
wherein the dry extract of lingonberry (*Vaccinium vitis-idaea* L.) has a drug/extract ratio (DEV) ranging from 65 : 1 to 40 : 1, preferably 60 : 1 to 43 : 1, more preferably 55 : 1 to 45 : 1, based on the dry extract, and/or
wherein component c) and/or the extract of lingonberry (*Vaccinium vitis-idaea* L.) has a proanthocyanidin content ranging from 0.05 to 3.5 wt.-%, preferably 0.1 to 3 wt.-%, more preferably 0.25 to 2.5 wt.-%, based on the total composition of the extract, and/or
wherein the dry extract of lingonberry (*Vaccinium vitis-idaea* L.) has a proanthocyanidin content ranging from 15 to 75 wt.-%, in particular 20 to 65 wt.-%, preferably 22.5 to 55 wt.-%, more preferably 25 to 50 wt.-%, based on the dry extract.

6. Composition according to any one of the preceding claims,
wherein the composition further comprises, as component e), pentylene glycol; and/or
wherein the composition further comprises, as component f), hyaluronan and/or physiologically compatible salts thereof, in particular physiologically compatible salts of hyaluronan, preferably sodium hyaluronate; and/or
wherein the composition further comprises, as component g), lactic acid and/or physiologically compatible salts thereof, in particular lactic acid; and/or
wherein the composition further comprises, as component h), a physiologically compatible antioxidant, fungicide and/or bactericide, in particular from the group of acetophenones, preferably from the group of hydroxyacetophenones, more preferably 4-hydroxyacetophenone; and/or
wherein the composition further comprises, as component i), a physiologically compatible thickening agent, in particular from the group of cellulose derivatives, preferably from the group of cellulose ethers, more preferably cellulose gum.

7. Composition according to any one of the preceding claims,
wherein the composition has a pH value ranging from of 2 to 7, in particular ranging from 2.5 to 6.75, preferably ranging from 2.75 to 6, more preferably ranging from 3 to 6.

8. Composition according to any one of the preceding claims,
wherein the composition is in the form of a cream, lotion, cream-gel and/or gel, in particular gel.

9. Composition according to any one of the preceding claims,
wherein the composition comprises at least one gelling agent,
in particular wherein the gelling agent is selected from component f) and/or component i).

10. Composition, in particular pharmaceutical and/or cosmetic composition, according to any one of the preceding claims, for use in the prophylactic and/or therapeutic treatment of infections and/or inflammatory diseases of the urinary tract;
for topical application to the external intimate area, in particular the external vaginal area, preferably in the region of the urethral opening, more preferably in the region of the female urethral opening, and/or in the region of the perineum, preferably in the region of the female perineum.

11. Pharmaceutical preparation, in particular cream, lotion, cream-gel, and/or gel, preferably gel, more preferably drug or medical device, comprising a composition according to any one of claims 1 to 9,
for topical application to the external genital area, in particular the external vaginal area, preferably in the region of the urethral opening, more preferably in the region of the female urethral opening, and/or in the region of the perineum, more preferably in the region of the female perineum.

12. Cosmetic preparation, in particular a cream, lotion, cream-gel and/or gel, preferably gel, comprising a composition according to any one of claims 1 to 9,
for topical application to the external genital area, in particular the external vaginal area, preferably in the area of the urethral opening, particularly in the area of the female urethral opening, and/or in the area of the perineum, particularly in the area of the female perineum.

13. Use of a cosmetic preparation according to Claim 12 in the field of intimate care for topical application to the external intimate area, in particular the external vaginal area, preferably in the area of the urethral opening, more preferably in the area of the female urethral opening, and/or in the area of the perineum, preferably in the area of the female perineum.

14. Dosing device, preferably dispenser, in particular for prefarably portion-wise dispensing or application, comprising a composition according to any one of claims 1 to 9 or a preparation according to claim 11 or 12.

## Revendications

1. Composition, en particulier composition pharmaceutique et/ou cosmétique, en particulier pour l'application topique dans la région intime externe, de préférence pour une utilisation dans le traitement prophylactique et/ou thérapeutique d'infections et/ou de maladies inflammatoires du tractus urogénital,
où la composition comprend, en combinaison et en quantités respectivement efficaces, en particulier respectivement pharmaceutiquement et/ou cosmétiquement efficaces,
a) du bisabolol,
b) au moins un extrait de canneberge commune (*Vaccinium oxycoccos*),
où l'extrait de canneberge commune (*Vaccinium* oxycoccos) est un extrait liquide,
où l'extrait liquide contient au moins un extrait sec de canneberge commune
(*Vaccinium* oxycoccos) et un solubilisant,
où l'extrait sec de canneberge commune (*Vaccinium oxycoccos*) présente un rapport drogue/extrait (DEV) dans une plage de 20 : 1 à 1 : 10, par rapport à l'extrait sec,
c) au moins un extrait d'airelle rouge (*Vaccinium vitis-idaea* L.),
où l'extrait d'airelle rouge (*Vaccinium vitis-idaea* L.) est un extrait liquide,
où l'extrait liquide contient au moins un extrait sec d'airelle rouge (*Vaccinium vitis-idaea* L.) et un solubilisant,
où l'extrait sec d'airelle rouge (*Vaccinium vitis-idaea* L.) présente un rapport drogue/extrait (DEV) dans une plage de 75 : 1 à 35 : 1, par rapport à l'extrait sec, et
où l'extrait sec d'airelle rouge (*Vaccinium* vitis-*idaea* L.) présente une teneur en proanthocyanidine dans une plage de 0,01 à 5% en poids, par rapport à la composition totale de l'extrait,
d) du mannose,
où la composition comprend, par rapport à la composition totale,
le composant a) en des quantités dans la plage de 0,01% en poids à 1,25% en poids,
le composant b) en des quantités dans la plage de 0,1% en poids à 5% en poids,
le composant c) en des quantités dans la plage de 0,1% en poids à 5% en poids,
le composant d) en des quantités inférieures à 1% en poids.

2. Composition selon la revendication 1,
où la composition comprend, par rapport à la composition totale, le composant d) en des quantités inférieures à 0,95% en poids, de préférence en des quantités inférieures à 0,9% en poids, de préférence en des quantités inférieures à 0,85% en poids, et/ou en des quantités dans la plage de 0,1% en poids à 0,99% en poids, en particulier en des quantités dans la plage de 0,2% en poids à 0,95% en poids, de préférence en des quantités dans la plage de 0,4% en poids à 0,9% en poids, de préférence en des quantités dans la plage de 0,5% en poids à 0,85% en poids.

3. Composition selon la revendication 1 ou 2,
où la composition comprend, par rapport à la composition totale,
le composant a) en des quantités dans la plage de 0,05% en poids à 0,75% en poids, de préférence en des quantités dans la plage de 0,075% en poids à 0,25% en poids, de préférence en des quantités dans la plage de 0,085% en poids à 0,15% en poids, et/ou
le composant b) en des quantités dans la plage de 0,25% en poids à 2,5% en poids, de préférence en des quantités dans la plage de 0,5% en poids à 1,75% en poids, de préférence en des quantités dans la plage de 0,75% en poids à 1,25% en poids, et/ou
le composant c) en des quantités dans la plage de 0,25% en poids à 2,5% en poids, de préférence en des quantités dans la plage de 0,5% en poids à 1,75% en poids, de préférence en des quantités dans la plage de 0,75% en poids à 1,25% en poids.

4. Composition selon l'une quelconque des revendications précédentes,
où l'extrait sec de canneberge commune (*Vaccinium oxycoccos*) est formulé et/ou présent conjointement avec et/ou en combinaison avec un excipient;
où l'excipient est un excipient soluble dans l'eau ou dispersible dans l'eau, en particulier à base et/ou contenant des glucides, de préférence à base et/ou contenant des oligo-saccharides et/ou des polysaccharides, de préférence est la maltodextrine, et/ou
où l'extrait sec contient l'excipient en des quantités dans une plage de 20 à 70% en poids, en particulier de 25 à 60% en poids, de préférence de 30 à 55% en poids, de préférence de 30 à 50% en poids, par rapport à l'extrait sec; et/ou
où l'extrait sec de canneberge commune (*Vaccinium oxycoccos*) présente un rapport drogue/extrait (DEV) dans une plage de 15 : 1 à 1 : 5, de préférence de 10 : 1 à 1 : 1, de préférence de 7 : 1 à 3 : 1, par rapport à l'extrait sec.

5. Composition selon l'une quelconque des revendications précédentes,
où l'extrait sec d'airelle rouge (*Vaccinium vitis-idaea* L.) présente un rapport drogue/extrait (DEV) dans une plage de 65 : 1 à 40 : 1, de préférence de 60 : 1 à 43 : 1, de préférence de 55 : 1 à 45 : 1, par rapport à l'extrait sec, et/ou
où le composant c) ou l'extrait d'airelle rouge (*Vaccinium vitis-idaea* L.) présente une teneur en proanthocyanidine dans une plage de 0,05 à 3,5% en poids, de préférence de 0,1 à 3% en poids, de préférence de 0,25 à 2,5% en poids, par rapport à la composition totale de l'extrait, et/ou
où l'extrait sec d'airelle rouge (*Vaccinium* vitis*-idaea* L.) présente une teneur en proanthocyanidine dans une plage de 15 à 75% en poids, en particulier de 20 à 65% en poids, de préférence de 22,5 à 55% en poids, de préférence de 25 à 50% en poids, par rapport à l'extrait sec.

6. Composition selon l'une quelconque des revendications précédentes,
où la composition comprend en outre comme composant e) du pentylène glycol; et/ou
où la composition comprend en outre comme composant f) de l'hyaluronane et/ou des sels physiologiquement acceptables de celui-ci, en particulier des sels physiologiquement acceptables d'hyaluronane, de préférence de l'hyaluronate de sodium; et/ou
où la composition comprend en outre comme composant g) de l'acide lactique et/ou des sels physiologiquement acceptables de celui-ci, en particulier de l'acide lactique; et/ou
où la composition comprend en outre comme composant h) un antioxydant, fongicide et/ou bactéricide physiologiquement acceptable, en particulier du groupe des acétophénones, de préférence du groupe des hydroxyacétophénones, de préférence de la 4-hydroxyacéto-phénone; et/ou
où la composition comprend en outre comme composant i) un épaississant physiologiquement acceptable, en particulier du groupe des dérivés de cellulose, de préférence du groupe des éthers de cellulose, de préférence de la gomme de cellulose.

7. Composition selon l'une quelconque des revendications précédentes,
où la composition présente un pH dans une plage de 2 à 7, en particulier dans une plage de 2,5 à 6,75, de préférence dans une plage de 2,75 à 6, de préférence dans une plage de 3 à 6.

8. Composition selon l'une quelconque des revendications précédentes,
où la composition est réalisée sous forme de crème, lotion, gel de crème et/ou gel, en particulier sous forme de gel.

9. Composition selon l'une quelconque des revendications précédentes,
où la composition comprend au moins un agent gélifiant,
en particulier où l'agent gélifiant est choisi parmi le composant f) et/ou le composant i).

10. Composition, en particulier composition pharmaceutique et/ou cosmétique, selon l'une quelconque des revendications précédentes, pour une utilisation dans le traitement prophylactique et/ou thérapeutique d'infections et/ou de maladies inflammatoires des voies urinaires;
pour l'application topique dans la région intime externe, en particulier dans la région vaginale externe, de préférence dans la région du méat uriniaire, de préférence dans la région du méat urinaire féminin, et/ou dans la région du périnée, de préférence dans la région du périnée féminin.

11. Préparation pharmaceutique, en particulier crème, lotion, gel de crème et/ou gel, de préférence gel, de préférence médicament ou dispositif médical, comprenant une composition selon l'une quelconque des revendications 1 à 9,
pour l'application topique dans la région intime externe, en particulier dans la région vaginale externe, de préférence dans la région du méat uriniaire, de préférence dans la région du méat urinaire féminin, et/ou dans la région du périnée, de préférence dans la région du périnée féminin.

12. Préparation cosmétique, en particulier crème, lotion, gel de crème et/ou gel, de préférence gel, comprenant une composition selon l'une quelconque des revendications 1 à 9,
pour l'application topique dans la région intime externe, en particulier dans la région vaginale externe, de préférence dans la région du méat uriniaire, de préférence dans la région du méat urinaire féminin, et/ou dans la région du périnée, de préférence dans la région du périnée féminin.

13. Utilisation d'une préparation cosmétique selon la revendication 12 dans le domaine des soins intimes,
pour l'application topique dans la région intime externe, en particulier dans la région vaginale externe, de préférence dans la région du méat uriniaire, de préférence dans la région du méat urinaire féminin, et/ou dans la région du périnée, de préférence dans la région du périnée féminin.

14. Dispositif de dosage, de préférence distributeur de dosage, en particulier pour la distribution ou l'application de préférence par portions, contenant une composition selon l'une quelconque des revendications 1 à 9 ou une préparation selon la revendication 11 ou 12.
